# EUROPEAN PATENT APPLICATION

(11) **EP 4 545 046 A1**
(43) Date of publication of application: **30.04.2025**
(21) Application number: 23020483.6
(22) Date of filing: 23.10.2023
(51) Int. Cl.: A61F 2/24

(54) **TRANSCATHETER TREATMENT OF ATRIOVENTRICULAR VALVES, TO TREAT LEAFLET PROLAPSE**

(71) Applicant: Verine, Vitali, 1207 Geneva (CH)
(72) Inventor: The designation of the inventor has not yet been filed

(57) **Abstract**

Method and apparatus for treating prolapse of an atrioventricular valve. An implant (30) is delivered by catheter (32) into the heart, the implant configured to attach to a native chordae tendineae (20) and to shorten the functional length of the native chordae tendineae.

## Description

### Field of the Invention

The present invention relates to the field of transcatheter treatment of atrio-ventricular cardiac valves. Some non-limiting aspects are directed to treating prolapse of native valve leaflets.

### Background to the Invention

The mammalian heart has two atrio-ventricular (A-V) valves which regulate blood flow internally between the atrium and ventricle. Correct atrio-ventricular valve function is important to maintain efficient pumping of blood by the heart. In a healthy heart, each atrio-ventricular valve opens during diastole to admit blood from the atrium to the ventricle, and closes during systole to prevent backflow. However, many patients suffer from atrio-ventricular valve insufficiency, when the leaflets fail to close properly, which is a life-degrading condition, and can be life-threatening. Blood regurgitation through the valve reduces the efficiency of the heart, requiring the heart muscle to work harder to compensate. Not only does regurgitation reduce flow of blood out of the heart circulating to organs, it exposes the atrium to an abnormal increase in blood pressure. Excessive atrial pressure can negatively impact the atria and incoming veins, and also obstruct free flow of blood into the heart.

Insufficiency of an atrio-ventricular valve can have many causes. A frequent cause is prolapse of one or both native leaflets. If the patient is suitable for open-heart surgery, prolapse can be treated by various surgical techniques, for example, mitral ring annuloplasty, chordae shortening by surgically burying the chordae in a papillary muscle trench, and more recently surgical implantation of artificial chordae. However, a large number of patients are not suitable for open heart surgery, for example, due to age or frailty or other risk factors and comorbidities.

Minimally invasive treatment of atrio-ventricular valve prolapse has long been sought after, but current technologies have significant limitations. Minimally invasive options include transcatheter implantation of a prosthetic replacement valve, and transcatheter implantation of devices that modify the native valve apparatus, such as leaflet clips, leaflet coaptation surfaces, artificial chordae and annuloplasty devices. However, current replacement atrio-ventricular valves are expensive, relatively large in size with special anchoring, and require a large diameter catheter that limits access to a transapical route. Leaflet clips and coaptation modifying devices restrict the ability of the valve to open fully, and so the treatment may have undesirable secondary effects. Artificial chordae are problematic because they have to be anchored to native tissue at either end which is difficult to achieve reliably using only catheter tools. Also, unlike native chordae, it is not possible to attach artificial chordae to mobile regions of the leaflet. Therefore, artificial chordae do not perform the full function of native chordae. Transcatheter annuloplasty devices can improve valve function within limits, but are best used in combination with another treatment.

Examples of some current techniques are described in US 2005/021057, US 2018/206992 and US 2007/255396.

### Summary of the invention

It would be desirable to address and/or mitigate one or more of the above issues.

Broadly speaking, a first aspect of the invention provides apparatus for treating prolapse of an atrioventricular valve, the apparatus comprising an implant deliverable by catheter, the implant configured to attach to a native chordae tendineae and to shorten the functional length of the chordae tendineae.

As used herein the term "chordae tendineae", or hereafter "chordae" refers to any of the chords of tissue that connect the native atrioventricular leaflets to the papillary muscles. Optionally, the implant is attachable to an individual native chordae (or individual native chord), and to shorten the functional length of the respective individual chordae. The term "chordae" can thus refer where appropriate to an individual chord, or multiple chords.

By shortening the functional length, an existing native chordae can better limit the range of movement of the native leaflet and enable leaflet prolapse to be corrected, without many of the limitations and inconveniences of prior art techniques discussed above. Shortening the functional length can also maintain or re-establish correct anatomical function of the or a native chordae, which is a consideration overlooked by many prior art techniques. For example, the native chordae can maintain or re-establish tension force between the native leaflets and the papillary muscles of the ventricle, which may be beneficial to heart function and to ventricle modelling behaviour.

Shortening the functional length of a chordae (e.g. an individual chordae) may especially include shortening the functional length independently of at least another chordae. Additionally or alternatively, shortening the functional length of a chordae (e.g. an individual chordae) associated with a first valve leaflet may especially include shortening the functional length independently of a chordae associated with a second valve leaflet that coapts with the first valve leaflet (e.g. an opposite leaflet of a bileaflet valve).

In some embodiments, the implant is configured to shorten the functional length by diverting a portion of the native chordae along a circuitous path. The circuitous path may optionally be a path into the implant, for example, into an interior cavity of the implant and/or into a space bound at least partly by the implant. Optionally, the implant defines at least partly the circuitous path along which the diverted portion of the native chordae extends, optionally at least a majority of the circuitous path, optionally substantially the entirety of the circuitous path. By using the implant to define the circuitous path, at least the majority of the diverted portion of the native chordae is controlled and supported by the implant, and not left loose to flap or flutter in an uncontrolled way, which may otherwise be unnatural and/or undesirable from a surgical point of view. The circuitous path defined by the implant may create, or correspond to creating, a fold or plication in the native chordae.

In other embodiments, the circuitous path may optionally be a path through the implant. For example, the implant may comprise a collar or ring or annulus through which a portion of the native chordae is pulled (or "doubled"). The pulled through portion enters and exits the collar (or ring or annulus) on the same side, with a doubled portion extending to the other side of the collar (or ring or annulus). The collar (or ring or annulus) may be configured to contract to secure the native chordae in a shortened state.

In some embodiments, the apparatus comprises a hook element for hooking the native chordae. The hook may form part of the implant or part of a delivery catheter for delivering the implant to a target site in the heart. The hook may be configured for pulling the native chordae in a proximal direction, the proximal direction being defined with respect to the catheter. Additionally or alternatively, the hook may be configured for pulling a portion of the native chordae, into and/or through a portion of the implant, in response to relative movement between the implant portion and the hook, for example, into and/or through (i) a cavity of the implant, and/or (ii) a space bounded by the implant.

In some embodiments, the hook element has a permanent hook configuration. In other embodiments, the hook element may be configured to change configuration. The hook element may change configuration between at least any two of: a hooking configuration for selectively hooking the native chordae; a non-hooking and/or release configuration for releasing (or permitting release of) the native chordae from hooking engagement; and a closed configuration in which a cavity of the hook element is closed. Hook elements with changeable configuration are described later below.

Additionally or alternatively to any of the above, in some embodiments, the apparatus comprises a puller element for pulling, from a first end of a space bounded by the implant, a portion of the native chordae into/and through the space from a second end of the space.

The puller may form part of the implant or part of a delivery catheter for delivering the implant to a target site in the heart. The puller may optionally be configured for pulling the native chordae in a proximal direction, the proximal direction being defined with respect to the catheter.

In some embodiments, the puller element has a hook configuration, for example, as described above. Additionally or alternatively, the puller element may be configured to change configuration. The puller element may change configuration between at least any two of: an engagement configuration for selectively hooking or admitting the native chordae; a release configuration for releasing (or permitting release of) the native chordae from engagement; and a closed configuration in which a cavity of the puller element is closed.

In some embodiments, the implant is configured to retain at least a portion of the pulled-in (or pulled-through) segment of chordae. For example, at least a portion of the implant may be configured to contract around the pulled-in (or pulled-through) portion. Additionally or alternatively, a retainer (e.g. at least one retainer element, optionally at least two retainer elements, optionally at least three retainer elements) may be configured to obstruct pulling-out of the pulled-in (or pulled-through) portion.

The retainer (e.g. retainer element) may comprise a projection that projects into the interior space and/or projects inwardly from a wall of the implant. The wall may be shaped as one or more of a ring, a collar, a sleeve, a tube (whether or not the wall has openings therein). Additionally or alternatively, the projection may be integrally formed with the wall. For example, the projection may be a tab or finger that (i) can fold into a clearance of and/or a plane of the wall from which the projection extends, and/or (ii) extends from a periphery of the wall.

In some embodiments, the implant may be configured to change configuration between a non-deployed configuration for delivery to a target site in the heart, and a deployed configuration for attaching to a native chordae.

For example, the implant may comprise a first portion and second portion, one portion being configured to deploy around or with respect to, and/or fold around, the other when the implant changes from the non-deployed configuration to the deployed configuration.

Additionally or alternatively, the implant may be configured to compress the native chordae tendineae and/or compress around the native chordae tendineae, when the implant changes from the non-deployed configuration to the deployed configuration.

In some embodiments, the implant comprises a curved surface configured for defining an at least partly curved circuitous path for the native chordae tendineae. Optionally, the curved surface defines a majority of the circuitous path as a curved path. Providing a curved path can provide atraumatic contact with the native chordae tissue, as well as adding to the path length.

Additionally or alternatively, the implant comprises an elongate clamping surface configured to provide spread clamping contact, spread along a length of a portion of a native chordae. The elongate clamping surface may optionally be any one or more of: curved; linear; curvilinear; defined by interlocking toothed profiles.

Additionally or alternatively to any of the above, the implant may have a streamlined shape, in some examples a bead-shape (optionally a spherical shape). A streamlined shape may reduce any tendency for the implant to be influenced by blood flow around the implant.

Additionally or alternatively, the implant may have a (e.g. maximum) transverse dimension (e.g. diameter) of less than 6mm, optionally less than 5mm, optionally less than 4mm. For example, the dimension may be in the range of 2 to 5mm, optionally 2 to 4mm, optionally 2-3mm.

Additionally or alternatively to any of the above, the implant may comprise, and/or be made of, one or more lightweight materials, such as one or more of: titanium; titanium-containing alloy; polyether ether ketone (PEEK); pyloric carbon. Lightweight materials may reduce loads on the native chordae, and may also reduce risk of the native chordae becoming totally disconnected at both ends even if a diseased or damaged native chordae should rupture at one point.

Additionally or alternatively to any of the above, the implant may comprise a wire mesh and/or braid structure. Such a structure can combine flexibility with a sufficiently strong predefined shape, for example in the deployed configuration, to remain securely and atraumatically attached to a native chordae.

Additionally or alternatively to any of the above, the implant may comprise one or more surfaces defining at least partly a channel for accommodating and pressing on a native chordae extending therethrough. At least one surface may comprise and/or be coated with and/or carry (e.g. as a liner), a compressible and/or flowable material able to conform at least partly around the profile of the native chordae while frictionally engaging against the native chordae. Example materials include silicone, polyurethane foam, and biocompatible rubbers. Such an arrangement can provide a secure yet atraumatic engagement with a native chordae.

A closely related second aspect of the invention provides an implant deliverable by catheter for treating prolapse of an atrioventricular valve (for example, by shortening a functional length of a native chordae), the implant comprising a hook for hooking a native chordae, the hook positioned internally with respect to a portion of the implant.

A closely related third aspect of the invention provides an implant deliverable by catheter for treating prolapse of an atrioventricular valve (for example, by shortening a functional length of a native chordae), the implant comprising a hook for hooking a native chordae, the hook being movable inside a portion of the implant.

A closely related fourth aspect of the invention provides an implant deliverable by catheter for treating prolapse of an atrioventricular valve (for example, by shortening a functional length of a native chordae), the implant comprising structure configured to divert a portion of the native chordae along a circuitous path, at least a majority of the circuitous path defined by the implant.

A closely related fifth aspect of the invention provides an implant deliverable by catheter for treating prolapse of an atrioventricular valve (for example, by shortening a functional length of a native chordae), the implant comprising a first portion and a second portion, the second portion being configured to deploy around and/or with respect to, the first portion when the implant changes from the non-deployed configuration to the deployed configuration.

A closely related sixth aspect of the invention provides an implant deliverable by catheter for treating prolapse of an atrioventricular valve (for example, by shortening a functional length of a native chordae), the implant comprising a first portion and a second portion, the second portion being configured to fold around and/or with respect to, the first portion when the implant changes from the non-deployed configuration to the deployed configuration.

A closely related seventh aspect of the invention provides an implant deliverable by catheter for treating prolapse of an atrioventricular valve (for example, by shortening a functional length of a native chordae), the implant comprising a structure defining a path for accommodating a native chordae, the structure configured to compress the path and/or compress around the path, when the implant changes from the non-deployed configuration to the deployed configuration.

A closely related eighth aspect of the invention provides an implant deliverable by catheter for treating prolapse of an atrioventricular valve (for example, by shortening a functional length of a native chordae), the implant comprising a structure defining a path for accommodating a native chordae, the structure configured to compress the native chordae tendineae and/or compress around the native chordae tendineae, when the implant changes from the non-deployed configuration to the deployed configuration.

A closely related ninth aspect of the invention provides an implant deliverable by catheter for treating prolapse of an atrioventricular valve (for example, by shortening a functional length of a native chordae), the implant comprising an elongate clamping surface configured to provide spread clamping contact along a native chordae tendineae, the elongate clamping surface being curved, linear or curvilinear.

A closely related tenth aspect of the invention provides an implant deliverable by catheter for treating prolapse of an atrioventricular valve (for example, by shortening a functional length of a native chordae), the implant comprising comprises a curved surface defining an at least partly curved circuitous path for receiving the native chordae tendineae. The curved surface may optionally define a majority of the circuitous path as a curved path.

A closely related eleventh aspect of the invention provides an implant deliverable by catheter for treating prolapse of an atrioventricular valve (for example, by shortening a functional length of a native chordae), the implant comprising a first part pivotally coupled to a second part, the first and second parts collectively defining a circuitous path for the native chordae. The portions of the first and second parts defining the circuitous path may optionally define surfaces that are curved, linear of curvilinear.

A closely related twelfth aspect of the invention provides an implant deliverable by catheter for treating prolapse of an atrioventricular valve (for example, by shortening a functional length of a native chordae), the implant comprising a structure defining a circuitous path for a native chordae that alternates between opposite portions and/or sides of the implant. For example, the structure may define a buckle-like structure for guiding a native chordae on a meandering and/or undulating path.

A closely related thirteenth aspect of the invention provides an implant deliverable by catheter for treating prolapse of an atrioventricular valve (for example, by shortening a functional length of a native chordae), the implant comprise first and second portions having confronting surfaces for trapping a native chordae therebetween, at least one of the confronting surfaces of the first and second portions comprising at least one projecting tooth, and at least the other confronting surface comprising a recess for accommodating the tooth. Optionally, the confronting surfaces of both of the first and second portions comprise interfitting toothed profiles, optionally interlocking toothed profiles. In some embodiments, the confronting surfaces are curved. The first and second portions may define a curved circuitous path that meanders around toothed profiles along the length of the curve.

A closely related fourteenth aspect of the invention provides an implant deliverable by catheter for treating prolapse of an atrioventricular valve (for example, by shortening a functional length of a native chordae), the implant comprising a loop structure. The loop structure may optionally comprise a movable gate for admitting a native chordae to an interior space of the loop structure. Additionally or alternatively, the loop structure may optionally be configured to fold around a portion of the implant.

A closely related fifteenth aspect of the invention provides apparatus for treating prolapse of an atrioventricular valve (for example, by shortening a functional length of at least one native chordae), the apparatus comprising a plurality of implants, each attachable to the or a native chordae, and a delivery catheter having an accommodation region at or near a distal end, for accommodating the plurality of implants together. In some embodiments, at least two of the plurality of implants may be deployed generally concurrently by the delivery catheter, for example, for attachment to the same native chordae. Additionally or alternatively, in other embodiments, the implants may be deployed individually, for example, for attachment to different respective chordae and/or to different respective chordae portions.

A closely related sixteenth aspect of the invention provides apparatus for treating prolapse of an atrioventricular valve (for example, by shortening a functional length of at least one native chordae), the apparatus comprising a delivery catheter packaged in sterile packaging, the delivery catheter pre-loaded with at least one implant deployable from the catheter, for example, for attachment to a native chordae. Optionally, the catheter is pre-loaded with a plurality of implants.

A closely related seventeenth aspect of the invention provides an implant deliverable by catheter for treating prolapse of an atrioventricular valve (for example, by shortening a functional length of a native chordae), the implant comprising a mesh and/or braid structure, the structure configured to change shape to define, at least partly, a circuitous path for a native chordae.

A closely related eighteenth aspect of the invention provides an implant deliverable by catheter for treating prolapse of an atrioventricular valve (for example, by shortening a functional length of a native chordae), the implant comprising at least one part having a shape that is concave in a first direction, and convex in a second direction generally perpendicular to the first direction.

A closely related nineteenth aspect of the invention provides an implant deliverable by catheter for treating prolapse of an atrioventricular valve (for example, by shortening a functional length of a native chordae), the implant comprising first and second parts that, in a deployed condition of the implant, curve around each other, optionally in first and second different directions (e.g. perpendicular directions). The implant may have a generally bead or bulb shape. The shape may optionally be generally spherical.

A closely related twentieth aspect of the invention provides an implant deliverable by catheter for treating prolapse of an atrioventricular valve (for example, by shortening a functional length of a native chordae), the implant having a generally bead shape, at least in a deployed configuration of the implant. The bead shape may optionally be generally spherical.

A closely related twenty-first aspect of the invention provides an implant deliverable by catheter for treating prolapse of an atrioventricular valve (for example, by shortening a functional length of a native chordae), the implant comprising, at least in a deployed configuration, one or more surfaces defining at least partly a channel for accommodating and pressing on a native chordae extending therethrough. At least one surface may comprise and/or be coated with and/or carry (e.g. as a liner), a compressible and/or flowable material able to conform at least partly around the profile of the native chordae while frictionally engaging against the native chordae. Example materials include silicone, polyurethane foam, and biocompatible rubbers. Such an arrangement can provide a secure yet atraumatic engagement with a native chordae.

A closely related twenty-second aspect of the invention provides an implant deliverable by catheter for treating prolapse of an atrioventricular valve (for example, by shortening a functional length of a native chordae), the implant comprising a spool element for winding a portion of the native chordae tendineae at least partly therearound.

The spool element may be configured to turn around a spool axis.

In some embodiments, the spool comprises an engagement profile for applying a winding torque to a native chordae tendineae, for winding the chordae tendineae at least partly around the spool. Additionally or alternatively, the spool may comprise a (e.g. second) engagement profile for retaining the spool in a set rotational orientation with respect to the native chordae tendineae and/or with respect to a non-rotating portion of the implant. At least one such engagement profile may comprise a resiliently biased lug.

A closely related twenty-fourth embodiment provides an implant deliverable by catheter for treating prolapse of an atrioventricular valve (for example, by shortening a functional length of a native chordae), the implant configured to draw a portion of the native chordae tendineae into and/or through and/or at least partly around a portion of the implant, the degree of shortening of the functional length being dependent on, optionally the same as, the length of the portion that is drawn in and/or drawn through and/or drawn around.

A closely related twenty-fifth embodiment provides an implant deliverable by catheter for treating prolapse of an atrioventricular valve (for example, by shortening a functional length of a native chordae), the implant configured to provide a variable degree of shortening of functional length of a native chordae tendineae, the degree of shortening corresponding to a length of native chordae that is drawn into and/or through and/or around the implant.

In some embodiments, the implant has a plurality of settable configurations, each providing a respective degree of shortening of the functional length of the native chordae tendineae.

A closely related twenty-sixth aspect of the invention provides an implant deliverable by catheter for treating prolapse of an atrioventricular valve (for example, by shortening a functional length of a native chordae), the implant comprising a contact surface for contacting a native chordae, the contact surface comprising porous material.

The porous material may be configured for promoting tissue ingrowth from the native chordae. Tissue ingrowth may increase integration and/or anchoring of the implant with the chordae. Additionally or alternatively, tissue ingrowth may reinforce the native chordae, which may for example be beneficial if the native chordae had previously stretched due to tissue weakness. Additionally or alternatively, the porous material may provide a generally irregular contact surface, for example, to increase frictional engagement between the chordae and the implant, and thereby assist with anchoring of the implant with the native chordae. Additionally or alternatively, the porous material may provide a generally atraumatic contact surface allowing firm engagement with the native chordae without substantially damaging the integrity of the native chordae tissue.

Examples of porous material may include one or more selected from: PET, PTFE, ePTFE, biocompatible fabric, polyurethane (e.g. open-cell and/or foamed polyurethane).

The pore size (e.g. mean pore size) is optionally less than 1mm, optionally less than 0.8mm, optionally less than 0.7mm, optionally less than 0.6mm, optionally less than 0.5mm, optionally less than 0.4mm, optionally less than 0.3mm, optionally less than or about 0.2mm.

In some embodiments, the implant comprises a structural member (for example, comprising generally non-porous material), the structural member carrying the porous material to provide the aforesaid contact surface for contacting a native chordae. The porous material may be in the form of and/or comprised in a component, e.g. a liner or contact member, provided at an interior surface and/or exterior surface of the structural member. The component be secured to the structural member by any suitable technique, for example, by one or more sutures, or by adhesive attachment, or welding or fusing of the liner to the structural member. The component may cover substantially the entirety of the interior and/or exterior surface of the structural member, or only a portion of the interior and/or exterior surface.

In some embodiments in which the implant defines a path through or into the implant for receiving a portion of a native chordae, the porous material (e.g. component comprising the porous material) may be provided along or around the path. The porous material may be provided on one or more interior facing surfaces of the implant.

A closely related twenty-seventh aspect of the invention provides an implant deliverable by catheter for treating prolapse of an atrioventricular valve (for example, by shortening a functional length of a native chordae), the implant configured to engage a native chordae in a manner to cause thickening and/or inflammation of the native chordae, optionally without damaging substantially the integrity of the tissue of the native chordae.

Causing thickening and/or inflammation of the chordae may be advantageous in enhancing the mechanical strength of the native chordae. This can be especially advantageous if the native chordae had previously stretched in length due to mechanical weakness.

This aspect may optionally be combined with the twenty-sixth aspect above, in which porous material may be used to provide a contact surface for contacting the native chordae tissue.

A closely related twenty-eighth aspect of the invention provides a method of treating an atrioventricular cardiac valve, the method comprising introducing an implant to a target side in a heart using a delivery catheter, and attaching the implant to a native chordae to functionally shorten the native chordae.

The catheter may be introduced using a transvascular route to the heart. In some embodiments, the transvascular route may be a transvenous transseptal route. In other embodiments, the transvascular route may be an arterial route. In other embodiments, the catheter may be introduced through a wall of the heart, for example, near or at the apex of the heart.

In some embodiments, the step of attaching comprises attaching the implant selectively to a primary chord (e.g. a primary native chordae). A primary chord is a chord that extends from a periphery of a valve leaflet, towards a papillary muscle, to control the peripheral edge. By attaching the implant selectively to a primary chord, it is possible adjust the peripheral edge, for example, to reduce or eliminate prolapse.

Additionally or alternatively, in some embodiments, the step of attaching the implant comprises attaching the implant selectively to a secondary chord (e.g. a secondary native chordae). A secondary chord is a chord that extends from a belly portion of a valve leaflet, towards a papillary muscle, to control bulging of the leaflet belly. By attaching the implant selectively to a secondary chord, it is possible to adjust leaflet bulging, for example, to reduce or eliminate prolapse.

Additionally or alternatively, in some embodiments, both a primary and a secondary chord may be treated by attachment of at least one implant (for example, at least one implant attached to both a primary chord and a secondary chord, or multiple implants attached at least one to at least one of a primary chord and secondary chord).

Additionally or alternatively to any of the above, the step of attaching may comprise attaching the implant to a native chordae at a position selected from (i) closer to the papillary muscle than to the leaflet; (ii) closer to the leaflet than to the papillary muscle; or (iii) generally midway between the leaflet and the papillary muscle.

A closely related twenty-ninth aspect of the invention provides a method of treating an atrioventricular cardiac valve, the method comprising:
observing a beating heart of a patient using medical imaging,
determining from the observation at least one target treatment characteristic relating to shortening of at least one native chordae for treating prolapse of one of more leaflets of an atrioventricular valve of the beating heart,
introducing a catheter to a target site in the beating heart of the patient, and
operating the catheter to attach an implant to a native chordae to functionally shorten the native chordae.

The step of observation may, for example, comprise using imaging such as trans-oesophageal ultra-sound imaging.

The treatment characteristic(s) may be any one or more of: a location of a target chordae to be shortened; a degree of shortening of a target chordae; a position on a target chordae at which to place an implant; a number of chordae on which to place one or more implants; a number of implants to be implanted.

The implant may be deployed from and/or via the catheter, to attach the implant to a native chordae.

The method may be effective to improve functioning of a dysfunctional atrioventricular heart valve. The method may be effect to correct, at least partly, prolapse of a valve leaflet.

A closely related thirtieth aspect of the invention provides a method of reinforcing a native chordae of an atrioventricular valve of a heart, the method comprising attaching an implant to the native chordae, the implant configured to reinforce a mechanical property of the native chordae by at least one of:
(i) Inducing inflammation of the tissue of the native chordae;
(ii) Inducing thickening of the tissue of the native chordae;
(iii) Inducing ingrowth of tissue into a porous contact surface of the implant.

In some embodiments, the step of attaching comprises attaching the implant without damaging the structural integrity of the tissue.

In some embodiments, the step of attaching comprises attaching the implant to tension the native chordae and/or reduce the functional length of the native chordae. For example, the step of attaching may comprise causing the implant to divert and/or secure, a portion of the native chordae in a circuitous, looped or folded state.

The foregoing aspects may be used independently of each other, or any two or more of the foregoing aspects may be combined together. All possible permutations and/or combinations of aspects are explicitly envisaged.

It will be appreciated from the above that the techniques of the invention can enable leaflet prolapse to be corrected while preserving native function of the sub-valvular apparatus. Shortening the functional length of a native chordae is also different from implanting an artificial chordae and adjusting the length of the artificial chordae during or after implantation. An artificial chordae is an artificial strand or filament that is less vulnerable to damage than would be a native chordae, especially a native chordae which has already stretched beyond its normal length resulting in leaflet prolapse. Artificial chordae are normally not attached to mobile leaflet regions, contrary to the native chordae. Special considerations apply in design of an implant for a native chordae, to avoid the implant from damaging or wearing the native tendon tissue of the chordae during the repeated opening and closing movements of the mobile leaflet portions from which native chordae extend.

Although certain features, ideas and advantages have been highlighted above, this is merely to aid understanding certain principles of the invention, without limiting the overall scope. Protection is claimed for any novel feature or idea described herein and/or illustrated in the drawings, whether or not emphasis has been placed thereon.

### Brief Description of the Drawings

Fig. 1 is a schematic sectional view of a heart ventricle illustrating a dysfunctional atrioventricular valve suffering from leaflet prolapse.
Fig. 2 is a schematic sectional view similar to Fig. 1, illustrating correction of prolapse by shortening the functional length of a native chordae tendineae.
Figs. 3a and 3b are schematic sectional views illustrating a first example of apparatus for treating prolapse.
Fig. 4 is a schematic sectional view showing the tip of the apparatus of Fig. 3 in more detail, carrying the implant in its non-deployed configuration, with the hook of the apparatus retracted ready to deploy the implant.
Fig. 5 is a schematic side view illustrating the implant of the first example in its deployed configuration attached to a native chordae.
Fig. 6 is a schematic sectional view of a second example of implant in its deployed configuration.
Fig. 7 is a schematic sectional view of a third example of implant.
Fig. 8 is a schematic plan view of a fourth example of implant.
Fig. 9 is a schematic side view of the fourth example of implant.
Fig. 10 is a schematic side view of a fifth example of implant in a deployed configuration.
Fig. 11 is a schematic side view of the fifth example of implant in a non-deployed configuration.
Fig. 12 is a schematic side view of the fifth example of implant attached to a native chordae.
Fig. 13 is a schematic side view of a sixth example of implant in a deployed configuration.
Fig. 14 is a schematic side view of a seventh example of implant in a non-deployed configuration.
Fig. 15 is a schematic side view of the seventh example of implant in a deployed configuration attached to a native chordae.
Fig. 16 is a schematic sectional view of an eighth example of implant carried by a catheter in a non-deployed configuration.
Fig. 17 is a schematic side view illustrating eversion of the implant between its different configurations.
Fig. 18 is a schematic sectional view of the eighth example of implant in its deployed configuration attached to a native chordae.
Fig. 19 is a schematic side view of a ninth example of implant in a non-deployed configuration.
Fig. 20 is a schematic side view of the ninth example of implant in a deployed configuration attached to a native chordae.
Fig. 21 is a schematic transverse view of the ninth example of implant.
Fig. 22 is a schematic side view of a tenth example of the implant in a non-deployed, open configuration, carried by a catheter
Fig. 23 is a schematic side view of the tenth example of the implant in a deployed, closed configuration, carried by a catheter.
Fig. 24 is a schematic sectional view similar to Fig. 23, but showing attachment to a native chordae.
Fig. 25 is a schematic side view of the tenth example of implant released from the catheter.
Fig. 26 is a schematic perspective view of an eleventh example of implant in a partly deployed (folded) configuration.
Fig. 27 is a schematic perspective view of the eleventh example in a non-deployed (unfolded) configuration.
Fig. 28 is a schematic sectional view of the eleventh example deployed (fully folded) configuration attached to a native chordae.
Fig. 29 is a schematic sectional view of a twelfth example of implant in a non-deployed configuration in a delivery catheter.
Fig. 30 is a schematic sectional view of the twelfth example attached to a native chordae.
Fig. 31 is a schematic sectional view of a thirteenth example of implants in a non-deployed configuration in a delivery catheter.
Fig. 32 is a schematic side view of a fourteenth example of implant comprising first and second parts nesting together.
Fig. 33 is a schematic sectional view of the fourteenth example of implant in a deployed configuration.
Fig. 34 is a schematic sectional view of the fourteenth example of implant in a non-deployed configuration attached to a delivery catheter.
Fig. 35 is a schematic sectional view of the fourteenth example when attached to a native chordae and released from the delivery catheter.
Fig. 36 is a schematic section view illustrating the delivery catheter for the fourteenth example.
Fig. 37 is a schematic sectional view of a fifteenth example of implant in a deployed configuration attached to a native chordae.
Fig. 38 is a schematic perspective view of a sixteenth example of implant with retainer projections.
Fig. 39 is a schematic side view of the implant of Fig. 38.
Fig. 40 is schematic side view illustrating a first deployment stage for the implant of Figs. 38 and 39.
Fig. 41 is a schematic side view illustrating a second deployment stage for the implant of Figs. 38 and 39.
Fig. 42 is a schematic side view of the implant attached to a native chordae.
Fig. 43 is a front perspective view of a seventeenth example of implant similar to the sixteenth example.
Fig. 44 is a schematic transparent side view showing a first deployment stage using the implant of Fig. 43.
Fig. 45 is a schematic transparent side view showing the implant attached to a native chordae.
Fig. 46 is a perspective view of a further configuration of hook element in an eighteen example.
Fig. 47 is a schematic side view showing how the hook element of Fig. 46 can cooperate with an implant.
Fig. 48 is a schematic side view illustrating a nineteenth example of implant with a single retainer projection extending from opposite the mouth.
Fig. 49 is a schematic side view illustrating a twentieth example of implant with a single retainer projection extending from adjacent to the mouth.
Fig. 50 is a schematic side view of a hook element of a twenty-first example, in which the hook element forms part of the implant.
Fig. 51 is a schematic side view similar to Fig. 50, but showing the hook element coupled to a catheter shaft.
Fig. 52 is a schematic cross-section of the assembled implant in an implanted state.
Fig. 53 is a schematic side view, similar to Fig. 50, but showing a modification in a twenty-second example.
Fig. 54 is a schematic side view similar to Fig. 53, but showing the hook element coupled to a catheter shaft.
Fig. 55 is a schematic cross section of the assembled implant in an implanted state.
Fig. 56 is a schematic cross-section view of a twenty-third example of implant carried on a delivery catheter.
Fig. 57 is a schematic cross-section view of the twenty-third example in a first stage during implantation to hook a native chordae.
Fig. 58 is a schematic cross-section view of the twenty-third example showing the implant body being advanced towards the puller.
Fig. 59 is a schematic cross-section view similar to Fig. 58, but showing the puller passing through the implant body.
Fig. 60 is a schematic view of the implant in its implanted state.
Fig. 61 is a schematic cross-sectional view similar to Fig. 56, but showing a modification in a twenty-fourth example.
Fig. 62 is a schematic cross-sectional view during the implantation procedure, with the puller parts pulled together.
Fig. 63 is a schematic view showing the implant in its implanted state and separated from the delivery catheter.
Fig. 64 is a schematic section view showing a variable configuration hook element in a twenty-fifth example described herein.
Fig. 65 is a schematic section view showing a modified configuration of hook element in a twenty-sixth example.
Fig. 66 is a schematic cross section view of a modified configuration of hook element in a twenty-seventh example, shown in a relaxed state.
Fig. 67 is a schematic cross-section view of the twenty-seventh embodiment in a tensioned state.
Fig. 68 is a schematic perspective view showing a hook element and concave member of a twenty-eighth example of implant.
Fig. 69 is a schematic perspective view of the implant in its implanted state.
Fig. 70 is a schematic perspective view from the side of an implant of a twenty-ninth example with a collapsible hub.
Fig. 71 is a schematic side view similar to Fig. 70, but with the hub expanded.
Fig. 72 is a top view of Fig. 71.
Fig. 73 is a schematic perspective view of thirtieth example in the form of a staple.
Fig. 74 is a schematic view of the staple of Fig. 73 after "stapling".
Fig. 75 is a schematic side view of a stapler unit integrated in a delivery catheter.
Fig. 76 is a schematic side view of a thirty-first example of implant, in the form of a spool element.
Fig. 77 is a schematic perspective view of the spool element of Fig. 76.
Fig. 78 is a schematic side view of the spool element mounted on a delivery catheter, illustrating a deployment technique for attaching to a native chordae.
Fig. 79 is a schematic side view of a thirty-second example of implant in the form of a spool element.
Fig. 80 is a schematic exploded view of components of a delivery catheter for the thirty-second example.
Fig. 81 is a schematic cross-section illustrating deployment of the implant from the delivery catheter.
Fig. 82 is a schematic exploded view of certain components of a thirty-third example of implant and delivery system, based on a spool element.
Fig. 83 is a schematic side view of additional components that work with the components of

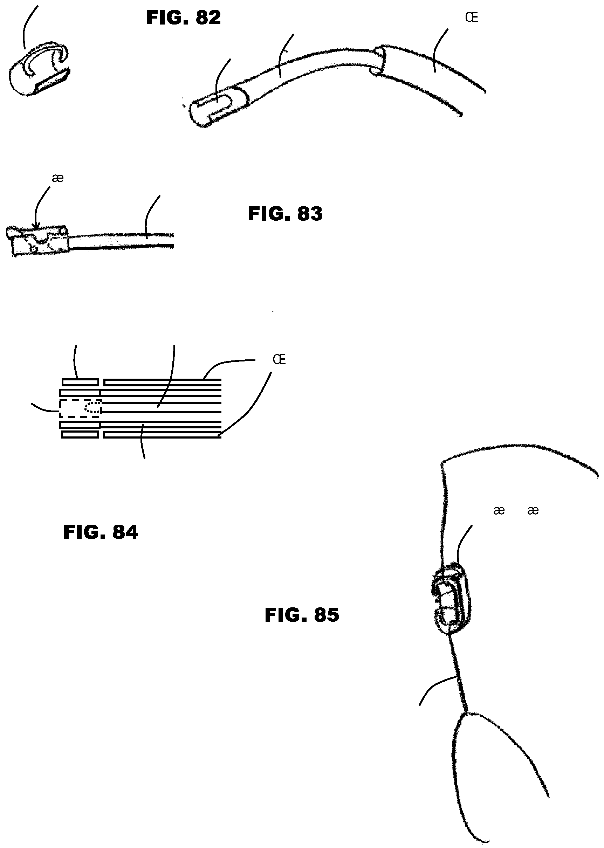

Fig. 82, including the spool element.
Fig. 84 is a schematic section illustrating a deployment stage during deployment of the implant.
Fig. 85 is a schematic section illustrating the implant attached to a native chordae.

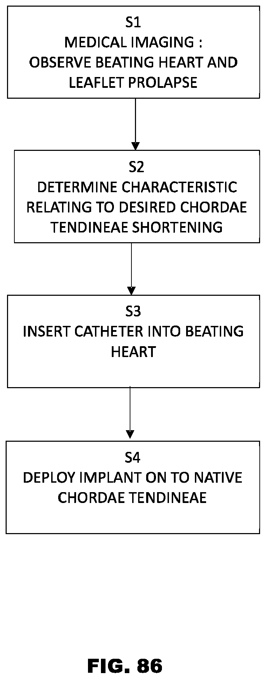

Fig. 86 is a schematic flow diagram illustrating treatment steps using the implant, in a thirty-fourth example.

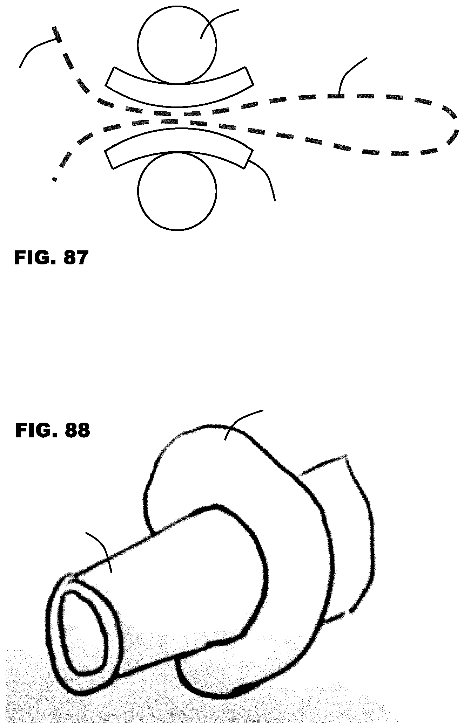

Fig. 87 is a schematic section illustrating a thirty-fifth example of implant, in the form of a torroid, and including a porous material component.
Fig. 88 is a schematic perspective view of the implant of Fig. 87 in isolation.

### Detailed Description of Preferred Embodiments

Non-limiting embodiments of the invention are now described by way of example, with reference to the accompanying drawings. In the drawings, the same or equivalent features are denoted by the same reference numerals whether or not described explicitly.

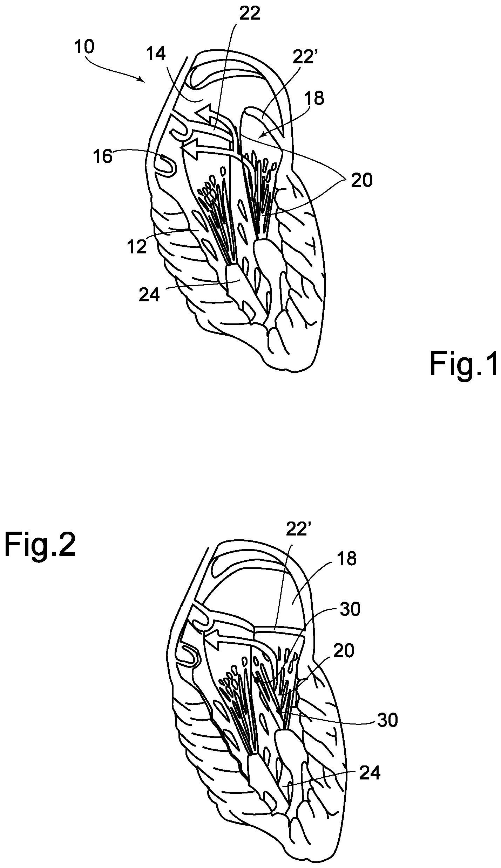

Referring to Fig. 1, a portion of a heart 10 is illustrated having a ventricle 12, an atrium 14, an outflow tract valve 16 leading to an artery from the heart, and an atrio-ventricular valve 18 between the ventricle 12 and atrium 14. The ventricle 12 may be a left ventricle, the outflow valve 16 an aortic valve, and the atrio-ventricular valve 18 a mitral valve. Alternatively, the ventricle 12 may be a right ventricle, the outflow valve 16 a pulmonary valve, and the atrio-ventricular valve 18 a tricuspid valve.

Chordae tendineae 20 (hereafter chordae) connect the leaflets 22 of the atrio-ventricular valve 18 to papillary muscles 24 in the ventricle 12, to control range of movement of the leaflets 22. In the example of Fig. 1, at least one leaflet 22' of the atrio-ventricular valve 18 is illustrated to suffer from prolapse, in which the chordae 20 do not restrain the leaflet 22' properly within the ventricle during systole, allowing the leaflet 22' to be pushed out of its correct position and at least partly into the atrium. Leaflet prolapse is a major cause of valve insufficiency and atrio-ventricular regurgitation, as illustrated by the white blood flow arrows.

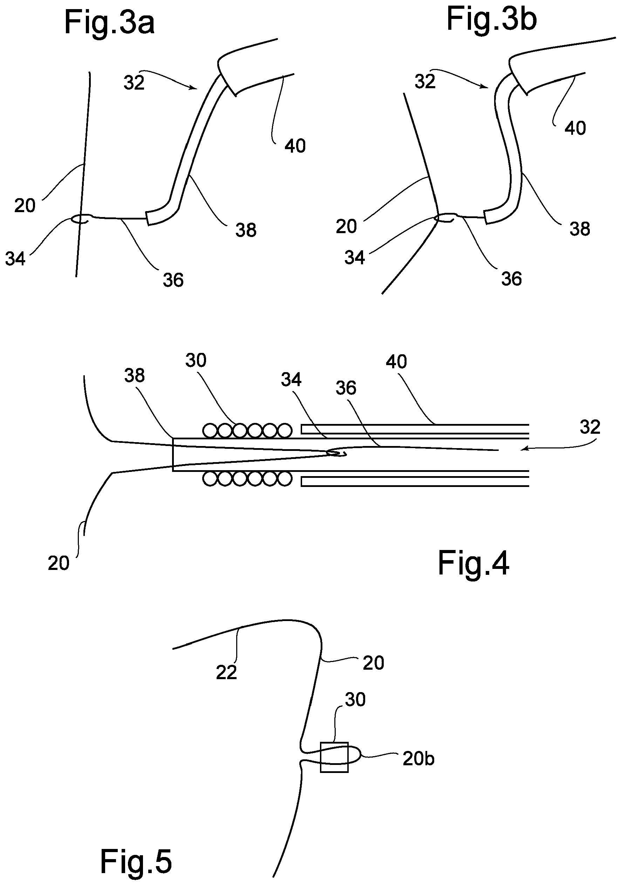

Figs. 2 to 4 illustrate one technique of the present invention to treat prolapse by a transcatheter procedure. An implant 30 (optionally multiple implants 30) is delivered by a delivery catheter (32 in Fig. 3) into the ventricle 12. The implant 30 is configured to attach to a native chordae 20, optionally an individual native chordae 20, to shorten the functional length of the native chordae 20. By shortening the functional length, the chordae 20 can better restrain the leaflet 22', correcting against prolapse, and restoring or improving valve function (as indicated by the white blood flow arrow in Fig. 2). The implant 30 and the delivery catheter 32 may be referred to as treatment apparatus.

The catheter may be introduced to a target site in the ventricle 12 and/or in the atrio-ventricular valve 18 by various access routes, including transvascular routes. For example, the catheter may be introduced via an arterial route through the outflow valve 16, for example, a transfemoral or transhumeral/transsubclavian route. Additionally or alternatively, the catheter may penetrate through the femoral vein and/or pass through the septum between left and right atria 14, namely a transseptal route.

Various examples of implant are described below. In some embodiments, the implant 30 is configured to change from a non-deployed configuration for delivery, to a deployed configuration for attachment to a native chordae. The implant 30 may be self-deploying when released from the delivery catheter, and/or the catheter may apply a deploying force to the implant to change the configuration. The implant 30 may optionally be made of a shape-memory material and/or super-elastic material, for example, a shape-memory alloy. One such alloy may comprise at least nickel and titanium, for example, nitinol. Other suitable materials include one or more of titanium, PEEK, silicone, and/or pyloric carbon.

In some examples, the implant comprises a contact surface or component comprising porous material 230 (Figs. 87 and 88). The porous material component 230 is illustrated explicitly in Figs. 87 and 88, but may be used with any of the other examples described herein, whether or not explicitly mentioned or illustrated.

The porous material may be configured for promoting tissue ingrowth from the native chordae. Tissue ingrowth may:
(i) increase integration and/or anchoring of the implant with the chordae; and/or
(ii) reinforce the native chordae, which may for example be beneficial if the native chordae had previously stretched due to tissue weakness; and/or
(iii) provide a generally irregular contact surface, for example, to increase frictional engagement between the chordae and the implant, and thereby assist with anchoring of the implant with the native chordae; and/or
(iv) provide a generally atraumatic contact surface allowing firm engagement with the native chordae without substantially damaging the integrity of the native chordae tissue.

Examples of porous material may include one or more selected from: PET, PTFE, ePTFE, biocompatible fabric, polyurethane (e.g. open-cell and/or foamed polyurethane).

The pore size (e.g. mean pore size) is optionally less than 1mm, optionally less than 0.8mm, optionally less than 0.7mm, optionally less than 0.6mm, optionally less than 0.5mm, optionally less than 0.4mm, optionally less than 0.3mm, optionally less than or about 0.2mm. In some examples, the implant comprises a structural member (for example, comprising generally non-porous material), the structural member carrying the porous material to provide the aforesaid contact surface for contacting a native chordae. The porous material may be in the form of and/or comprised in a component, e.g. a liner or contact member, provided at an interior surface and/or exterior surface of the structural member. The component be secured to the structural member by any suitable technique, for example, by one or more sutures, or by adhesive attachment, or welding or fusing of the liner to the structural member. The component may cover substantially the entirety of the interior and/or exterior surface of the structural member, or only a portion of the interior and/or exterior surface.

In some examples in which the implant defines a path through or into the implant for receiving a portion of a native chordae, the porous material (e.g. component comprising the porous material) may be provided along or around the path. The porous material may be provided on one or more interior facing surfaces of the implant.

Whether or not the porous material is provided, the implant 30 may define a circuitous path that shortens the function length of a native chordae by at least 1mm, optionally at least 2mm, optionally at least 3mm, optionally at least 4mm, optionally at least or about 5mm, optionally at least 10mm, optionally 15-20 mm in some cases. Optionally, multiple implants 30 may be attached to the same chordae to accumulate the shortening effects and achieve greater degrees of shortening than a single implant. In some embodiments, the implant is lightweight so as to avoiding adding significant load to the native chordae, especially should a diseased chordae rupture at one point. Also, in some embodiments, the implant has a streamlined profile in the implanted or deployed configuration.

The implant 30 may optionally be supplied pre-attached to, or pre-loaded in, the delivery catheter as a sterile assembly. The assembly may optionally be supplied packaged in sterile packaging. Alternatively, the implant may be supplied separately from the catheter, and attached to and/or loaded in the catheter shortly before the patient procedure.

In some embodiments, the apparatus comprises a puller element, for example, in the form of a hook (e.g. 34). The puller element may form part of the catheter and/or part of the implant. The puller element may be configured for pulling a portion of a native chordae: (i) in a proximal direction with respect to the catheter; and/or (ii) into and/or through an interior cavity or space bounded by the implant; and/or (iii) from a first end of the implant through a mouth of the implant at a second (e.g. opposite) end of the implant.

A hook configuration of puller element can provide advantages in being able to engage around a portion of a native chordae for pulling on the native chordae with confidence that the chordae will not accidentally slip out of engagement with the hook. A hook is illustrated in the following embodiments, although it is to be understood that a hook may be replaced by other forms of pulling element according to the desired implementation.

It is believed that, in addition to shortening the functional length of a native chordae, the implant 30 can be effective in reinforcing mechanical properties (e.g. strength) of the native chordae tissue. Reinforcement may be especially beneficial if the native chordae had previously elongated due to tissue weakness. Reinforcement may prevent, or at least slow, any tendency of the native chordae to further elongate after attachment of the implant. Reinforcement may occur as a result of the implant inducing one or more of:
(i) inflammation of the tissue of the native chordae;
(ii) thickening of the tissue of the native chordae;
(iii) ingrowth of tissue into a porous contact surface of the implant

Referring to Figs. 3 to 5, in a first example, the catheter 32 may comprise a hook 34 configured for hooking a native chordae 20 to pull the native chordae 20 proximally, ie. in a proximal direction of, or with respect to, the catheter 32. In the illustrated example, the catheter comprises a plurality of tubes nested one within another. The hook 34 is carried by a shaft 36 that is extendable from and/or retractable into the interior bore of a hollow carrier tube 38 carrying the implant 30. The carrier tube 38 is itself slidable within the bore of a pusher tube 40. In this example, the implant 30 has the form of a narrow-bore elongate coil. In a non-deployed configuration, the coil mounted outside the carrier tube 38 is held in a widened state. When released, the coil self-contracts to its narrow bore, deployed state.

In use (Fig. 3a), the catheter 32 is advanced to a target site in the vicinity of the native chordae 20 to be shortened, and the hook 34 is extended to hook the native chordae 20. The hook 32 is retracted (fig. 3b) to divert a portion 20a of the hooked chordae 20 towards the carrier tube, and ultimately into the carrier tube 38 (Fig. 4). Relative retraction of the carrier tube 38 and/or advancement of the pusher tube 40 pushes the coil implant 30 progressively off the free end of the carrier tube 38 and on to the chordae portion 20a.

Referring to Fig. 5, the implant coil 30 contracts towards its narrow bore configuration, clamping the chordae portion 20a within its interior. A portion 20b of the chordae forms a free loop at one end of the coil implant 30, but a majority of the chordae portion 20a is trapped in a circuitous hairpin path defined by the bore of the coil implant 30. The clamping force is distributed over a relatively long majority of the chordae portion 20a.

Figs. 87 and 88 illustrate a similar embodiment to Figs. 3-5, but in which the implant 30 has the form of a collar (or ring or torroid) that contracts around the pulled-through portion 20b of the native chordae, to secure the chordae in its shortened state. In a similar manner to that described above, the implant 30 is initially held in an expanded state by a catheter shaft, allowing a portion of the chordae to be hooked and pulled through the implant 30. The implant 30 self-contracts when released from the shaft.

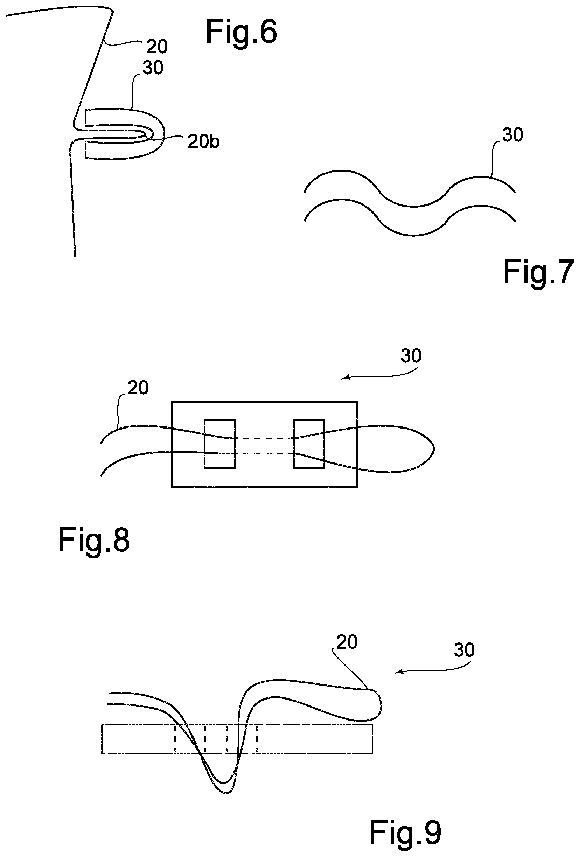

Referring to Fig. 6, a second embodiment of implant 30 similar to the first example, comprises a self-closing clip that traps at least a majority, optionally substantially the entirety, of the diverted chordae portion 20a with its interior. The interior defines a circuitous path also in the form of a hairpin.

Although in Figs. 5 and 6, the implant has a generally straight configuration, referring to Fig. 7, a third example of implant 30 may have a curvilinear configuration to define a wavy and/or tortuous path within the implant 30. A tortuous path can provide a longer length of circuitous path, and hence increase the shortening effect of the implant 30, as well as help prevent the chordae portion 20a from slipping out of the implant 30.

Figs. 8 and 9 illustrate a fourth example similar to Fig. 7, except that the implant 30 has a buckle profile, through which a native chordae can be pulled. Figs. 8 and 9 illustrate a folded chordae pulled through creating a double pass. The buckle like implant 30 could be attached to the chordae using the same principle shown in the Fig. 4.

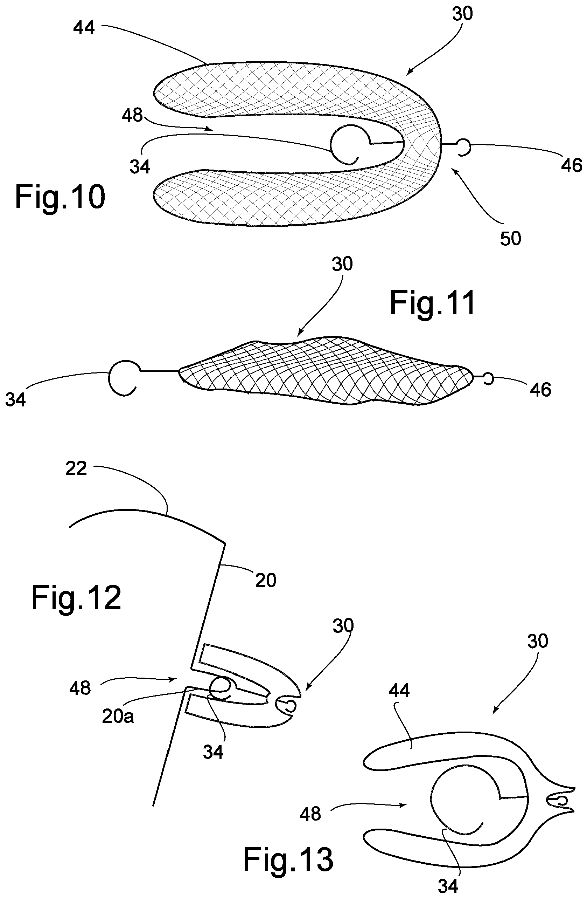

Figs. 10-12 illustrate a fifth example of implant 30 in which the hook 34 forms part of the implant. The implant comprises a (e.g. continuous) wire mesh 44 made, for example, of shape-memory material. The hook 34 is integrally mounted to one side, and a handle or grip 46 in the form of a secondary hook (here shown as an example while other connecting/disconnecting principles could be used) is mounted generally opposite. The handle (e.g. secondary hook) 46 enables the implant 30 to be attached to and manipulated by the catheter 32. Alternatively, the implant comprises a continuous wireform loop.

In its natural, deployed configuration (Fig. 10), the wire mesh and/or wireform loop defines a cup and/or channel 48 around the hook 34, with the hook 34 generally at the base of the cup/channel 48. The secondary hook 46 can optionally be accommodated in a smaller recess 50 intended to shroud any sharp or abrupt edges of the secondary hook 46 (figs. 12 and 13).

In use, the implant 30 is unfolded to a generally low profile, elongated, non-deployed configuration (Fig. 11) in which the implant is delivered to a target site by a catheter (not shown). The implant 30 is restrained in the non-deployed configuration by means of a constraining sheath, for example. In the non-deployed configuration, the hook 34 is exposed for hooking on to a native chordae portion 20a.

Referring to Fig. 12, when the constraining sheath is removed, the implant 30 reverts to its deployed configuration, deploying and/or folding around the hook 34 and pulling the native chordae portion 20 to divert the chordae portion 20a into the cavity of the channel 48, thereby shortening the functional length of the chordae 20.

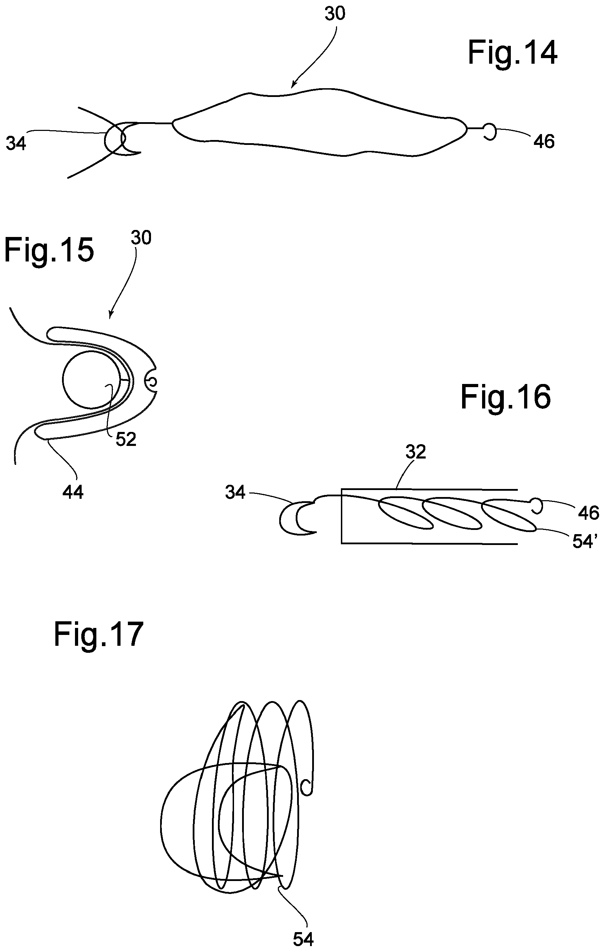

Figs. 13 illustrates a sixth example similar to the fifth example, but in which the wire mesh and/or wireform 44 has a more streamlined or curved shape folded around the hook 34, and defining a narrower and more rounded mouth to the channel 48. In Figs. 12 and 13, and also Figs. 14-15 described below, the mesh detail is omitted to avoid cluttering the drawing.

Figs. 14 and 15 illustrate a seventh example similar to the fifth and sixth examples above, but in which the hook 34 has a three-dimensional profile. For example, the hook 34 provides a concave hook recess in a lateral and/or transverse direction to allow lateral/transverse hooking of a chordae (Fig. 14). Additionally, the hook 34 has a convex profile in a perpendicular direction to define a curved hub surface 52 (Fig. 15). Alternatively, the hook 34 may be configured to change configuration as the implant 30 changes from the non-deployed configuration (Fig. 14) to the deployed configuration (Fig. 15). The hook 34 has arms that close to form a curved hub surface 52 around which the deviated chordae portion 20a extends, and around which the wire mesh and/or wireform 44 of the implant 30 folds. By using a curved surface 52, the circuitous path around the hub can be lengthened compared to a straight hairpin path. Also, the chordae is clamped over a relatively long contact surface. Additionally or alternatively, the central portion of the hook 34 could be made thicker as compared to the hooks from Figs. 10 to 13, to achieve a similar effect.

Figs. 16-18 illustrate an eighth example of implant 30 comprising a hook 34 similar to the hook of the sixth example, having a three-dimensional profile and/or configured to change configuration to a curved hub surface 52 when the implant is deployed. A coiled shell 54 extends from and cups around the hook/hub surface 52 to define a circuitous deviation path for a portion of a native chordae.

In the non-deployed configuration of the implant 30, the coiled shell 54 is pulled proximally away from the hook 34 to an everted condition 54', as illustrated in Fig. 16, and constrained within a sheath of the delivery catheter 32. The hook 34 is exposed to facilitate hooking on a native chordae.

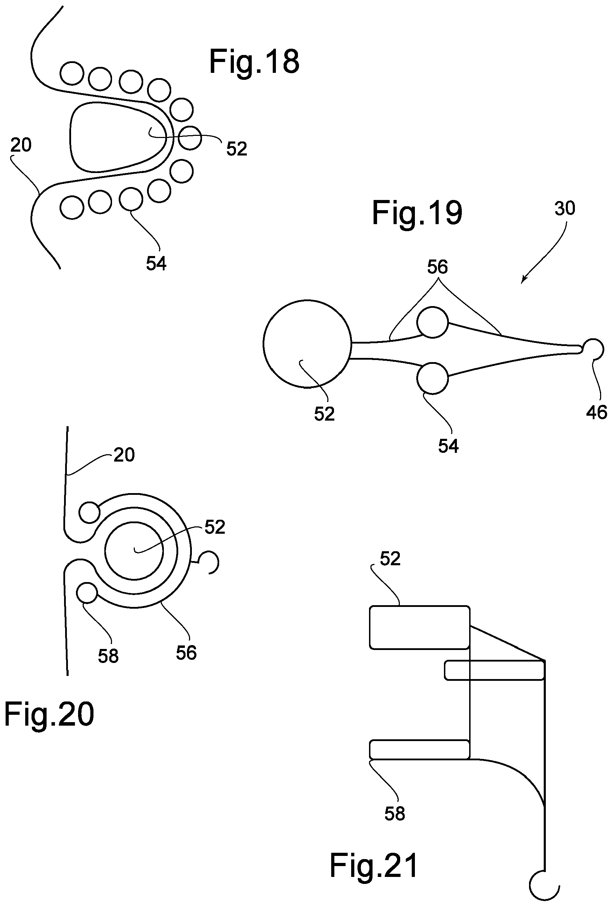

As depicted schematically in Fig. 17, when the everted coil 54' is released from the sheath, the coil 54 reverts distally to its original configuration. Referring to Fig. 18, the coil 54 cups around the hook 32/hub 52, and clamps the native chordae in a circuitous path extending around the curved surface of the hub 52.

Figs. 19-21 illustrate an ninth example of implant 30 comprising a hub 52 to which are coupled first and second arms 56 carrying lobes or posts 58. As can be seen in Fig. 21, the hub 52 and the lobes/fingers 58 have a transverse dimension greater than that of the arms 56, to define surfaces over which a native chordae is guided on a circuitous path. In a non-deployed configuration, the arms 56 are pulled proximally away from the hub 52 into a delivery catheter sheath (not shown), allowing the hub 52 to be hooked around a native chordae 20. When the implant is released, the arms 56 fold with respect to the hub 52, e.g. fold around the hub 52, such that the posts 58 divert the native chordae on a circuitous path around the hub 52. The implant further comprises a secondary hook 46 similar to the preceding embodiments.

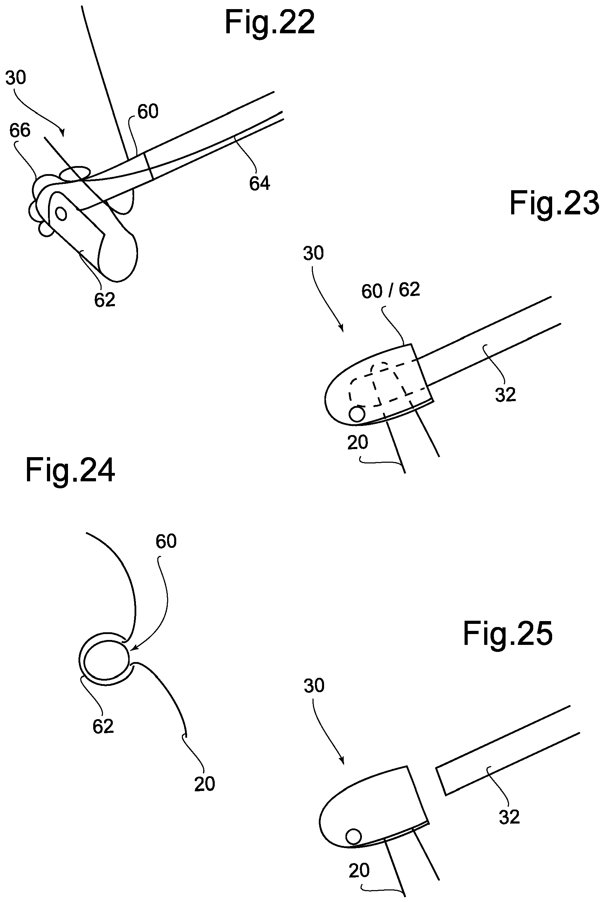

Figs. 22-25 illustrate a tenth example of implant 30 comprising first and second pivoting parts 60 and 62 controlled by means of an actuator 64 of the delivery catheter 32. The first and second parts 60 and 62 are arranged one nested within another, and have matching curved profiles to define a circuitous path therebetween around the tip of the inner part. The inner part acts as a curved head around which the outer part can pivot. In a non-deployed configuration of the implant 30, for delivery to a target site (Fig. 22), the outer part (e.g. the second part 62) is pivoted open away from the inner part to allow a portion of a native chordae to enter between the two parts. By actuating the implant 30 into the deployed configuration (Figs. 23 and 24), the outer part pivots into close engagement with the inner part, thereby diverting the chordae along the circuitous path defined between the two parts 60 and 62. The first and second parts 60 and 62 can be retained in the deployed configuration by any of: friction; and/or a resilient bias (e.g. spring 66 in Fig. 22); and/or a locking mechanism (not shown). After the implant 30 has been attached to the native chordae 20, the delivery catheter 32 is detached from the implant and withdrawn (Fig. 25).

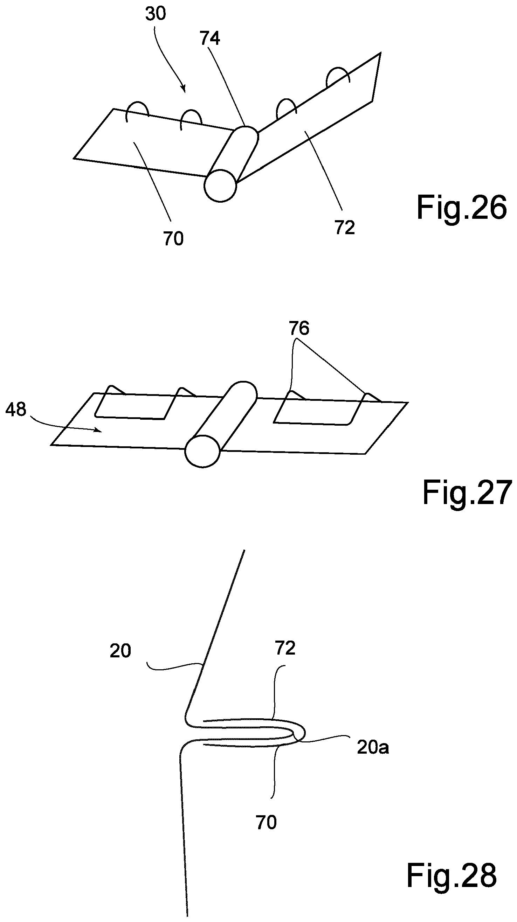

Figs. 26-28 illustrate an eleventh example of implant 30 comprising first and second limbs 70 and 72 that pivot relative to each other about a transverse hinge axis 74. The limbs 70 and 72, and/or trapping fingers 76 carried by the limbs 70 also fold over in a longitudinal direction generally perpendicular to the hinge axis 74 to define a clamping channel for receiving the native chordae 20. Once clamped, the first and second limbs 70 and 72 are pivot closed like jaws, to define a multiple-folded clamp (Fig. 28), defining a generally hairpin circuitous path.

The implant 30 may be held in the deployed configuration by any of: friction; and/or a resilient bias (not shown); and/or a locking mechanism (not shown).

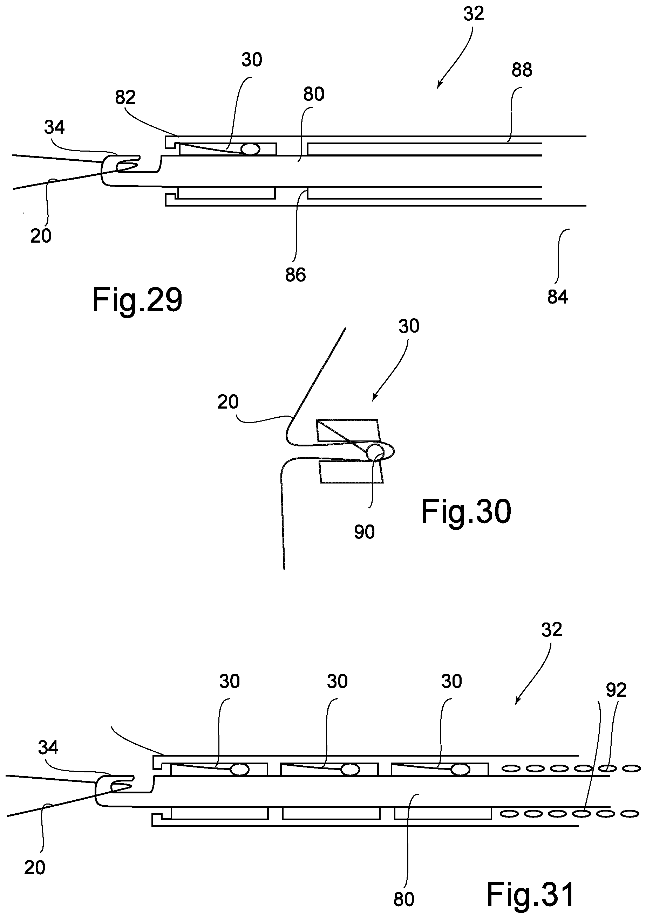

Figs. 29 and 30 illustrate a twelfth example of implant 30 and delivery catheter 32, which are similar in some respects to those of the first example (Figs. 3-5). The implant 30 comprises a self-contracting tube or coil configured to self-contract from an expanded condition for delivery (Fig. 29) to a contracted configuration (30) in which the tube accommodates a portion of a native chordae 20 in a hairpin shape. Also, the delivery catheter 32 comprises a hook 34 that is extendable to hook on a native chordae 20 (Fig. 29), and retractable to pull the native chordae proximally into the implant 30.

However, the twelfth example has additional features. Referring to Fig. 29, instead of requiring a carrier tube to hold the implant 30 in its expanded configuration for delivery, the implant 30 is supported directly by a shaft 80 on which the hook 34 is mounted. The shaft 80 is dimensioned to hold the implant 30 in its expanded non-deployed configuration for delivery. The shaft 80 is extendable to expose the hook 34, and retractable to pull the hook 34 into the interior of the catheter 32. The catheter 32 further includes stops to restrain the implant 30 axially during movement of the shaft 80, despite friction between the implant 30 and the shaft 80. In this example, a distal stop comprises an inwardly turned lip 82 of an outer cover tube 84, and a proximal stop comprises a blocking end surface 86 of an intermediate tube 88 within which the shaft 80 slides.

In use, the shaft 80 is extended to expose the hook 42 for hooking a native chordae 20. The shaft 80 is then retracted to pull the native chordae into the interior of the catheter 32. Once the shaft 80 and hook 42 have cleared the implant 30, the implant 30 is free to contract to its deployed configuration (Fig. 30) to attach around the portion of native chordae that has been pulled through the implant. The deployed configuration has a smaller diameter in which the implant is no longer restrained by the lip 82, and so is free to separate from the catheter 32. The catheter 32 can be withdrawn away from the deployed implant 30, or the shaft 80 can be advanced distally to eject the implant 30 using the hook 34 as a pusher.

A further feature of the implant 30 of this example is provision of a self-deploying projection 90 (e.g. acting as a retainer). In the non-deployed configuration (Fig. 29), the projection 90 is folded into the wall of the implant 30. In the deployed configuration (Fig. 30), the projection 90 deploys towards and/or across the interior passage of the implant 30, to define a surface behind the tight bend of the native chordae, thereby retaining the pulled-in portion of the chordae 20, and/or preventing the native chordae 20 from pulling out of the implant once the projection 90 has deployed. In the illustrated example, the projection 90 extends diagonally from one end. In an alternative embodiment, the projection could swing more transversely from the opposite end.

Fig. 31 illustrates a thirteenth example similar to the twelfth example above. The main difference is that the catheter is configured to accommodate multiple implants 30 loaded in line with each other at the tip. The implants 30 are deployed by manipulation of the shaft 80. For example, multiple implants 30 may be attached to the same native chordae 20 by retracting the shaft 80 sufficiently proximally to clear the appropriate number of implants 30 to be deployed together. Alternatively, each implant 30 can be deployed individually by retracting the shaft 80 only so far to clear the distal-most implant 30. The distal-most implant 30 contracts on to the chordae 20 and separates from the catheter 32, while the other implants 30 remain stocked within the catheter 32 and held in the non-deployed condition by the shaft 80. A spring-biased pusher 92, for example, a coil spring, urges the implants 30 distally to replace the deployed implant at the tip ready for a subsequent deployment. Such a catheter 32 allows multiple implants 30 to be attached, without having to withdraw and reinsert the catheter each time. Such a multiple use catheter 32 can greatly increase the versatility of use, and reduce the duration and invasiveness of the procedure.

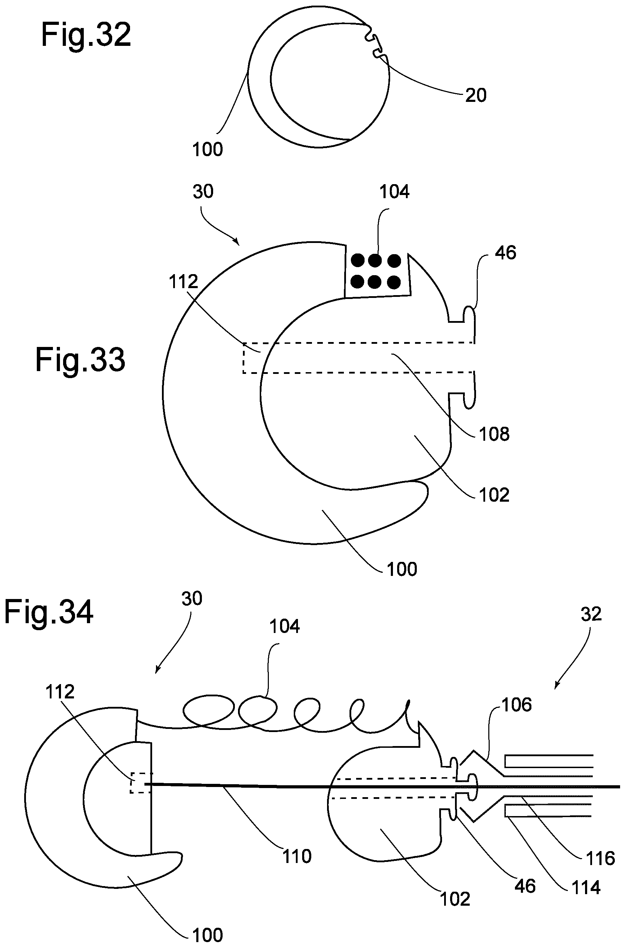

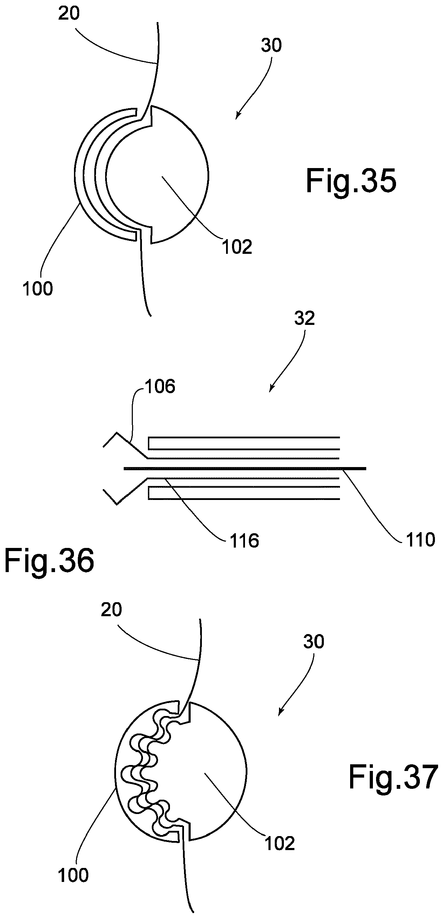

Figs. 32 to 36 illustrate a fourteenth example similar to the tenth example above, in that the implant 30 comprises complementary first and second parts 100 and 102 having curved profiles to define a circuitous path therebetween. In the present example, the first and second parts 100 and 102 are movable slidably with respect to each other. A spring 104 biases the parts 100 and 102 towards each other into the deployed configuration trapping a portion of the native chordae 20 between the parts 100, 102 (Fig. 35).

One part 102 comprises a handle 46 in the form of a nipple that can be gripped by gripper jaws 106 of the catheter 32, and a through bore 108 through which a pusher rod 110 of the catheter 32 can be advanced to push the parts 100 and 102 apart. The other part 100 optionally comprises a socket 112 for receiving the tip of the pusher rod 110. The through bore 108 is aligned centrally with the nipple 46. The catheter 32 comprises an outer sleeve 114 within which a hollow tube 116 carrying the gripper jaws 106 is slidable. The gripper jaws 106 are biased open but, by retracting the tube 116 with respect to the sleeve 114, the jaws 106 close inwardly to enable the nipple 46 to be gripped by the catheter 32 until the implant 30 is ready to be released.

The pusher rod 110 is slidable within the bore of the tube 116. In use, by advancing the pusher rod 110 while holding the tube 116 retracted to grip the nipple 46, the parts 100 and 102 of the implant are pushed apart against the bias of the spring 104. To attach the implant 30 to a native chordae 20, the parts 100 and 102 are hooked around the native chordae 20, and the pusher rod 110 retracted. The spring 104 pulls the parts 100 and 102 towards each other, to trap the native chordae 20 in a circuitous path between the curved profiles of the parts 100 and 102. Should the operator physician desired to adjust the position of the implant 30, this is possible while the implant 30 is still attached to the jaws 106. The pusher rod 110 may be advanced again to open the parts 100 and 102, and allow repositioning. Once the implant 30 has been correctly positioned and firmly attached, the tube 116 can be advanced to allow the jaws 106 to spring open, and release the nipple 46 of the implant. Thereafter, the catheter is withdrawn.

As best seen comparing Figs. 32 and 35, in the illustrated example, the parts 100 and 102 define a bulb or ball shape. The parts 100 and 102 may curve around each other in different, e.g. generally perpendicular, directions, for example, in a similar manner to the curvilinear surface contouring of a tennis ball, or in a similar manner to a person crossing the palms of his or her hands together. Such a form can assist in self-alignment of the parts 100 and 102 as they come together, while also providing a smooth outer surface. It can also provide complementary hook-style surfaces on both parts 100 and 102, acting in different directions. In other examples, the parts 100 and 102 could have more of a male-female configuration, such that one part 102 fits into the convex hollow of the other part 100.

In the fourteenth example, the confronting surfaces of the first and second parts 100 and 102 are generally smoothly curved with a large radius of curvature defining the circuitous path.

Fig. 37 illustrates a fifteenth example in which the confronting surfaces have interlocking and/or toothed profiles. Such profiles increase the length of the circuitous path, by additional meandering, compared to smooth profiles. Additionally or alternatively, the profiles can define a snap-fit engagement. Snap-fitting may be due to the angle of the teeth not all being aligned with the direction of movement. Alternatively, the some of the teeth may be configured to be oversize with respect to the mouth of the confronting recess, thereby forming a snap-fit as the teeth ride into the recesses. Snap-fitting can add a further degree of security to the attachment already provided by the spring 104.

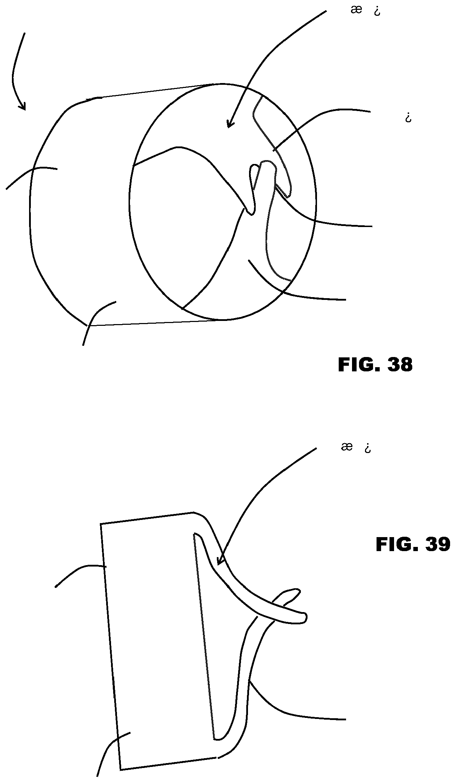

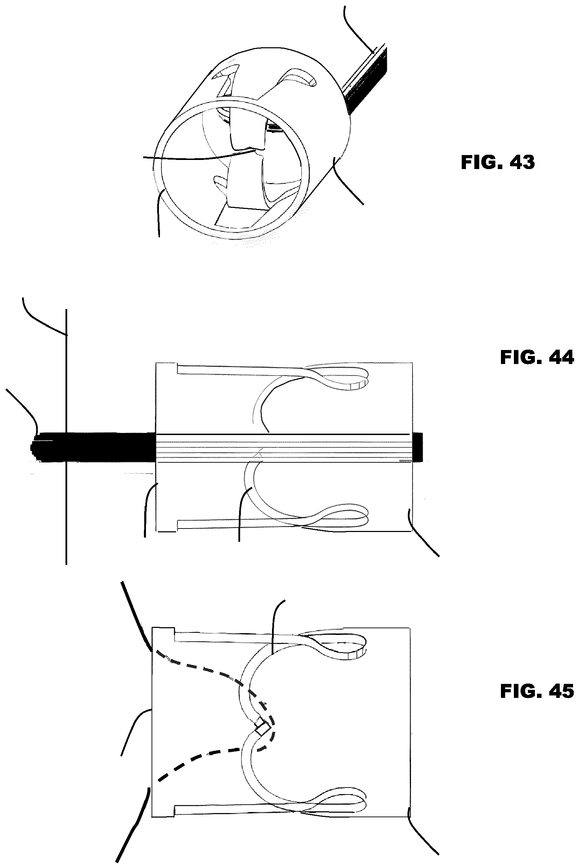

Figs. 38 to 49 illustrate further examples using a retainer 90 similar in function to that of the twelfth embodiment described above. The implant 30 comprises a collar (or sleeve or tubular) body 120 having a mouth 122 at one axial end, and a retainer 90. In the sixteenth example of Figs. 38 to 42, the retainer 90 is disposed at an opposite axial end of the body 120 to the mouth 122. Such a configuration can enable a relatively short implant body to be achieved. In the seventeenth to twentieth examples of Figs. 43 to 49, the retainer is disposed at an intermediate position between the axial ends of the body 120. Such a configuration can enable the retainer 90 to be shrouded by the body 120, and avoid the retainer 90 being exposed, if desired.

In these examples, the retainer 90 may comprise one or more projections that project, or bend, or are inclined, inwardly (e.g. radially inwardly) from the wall of the body 120. In the current examples, the projections are integrally formed with the wall of the body. Integral-forming can facilitate manufacture and a compact size. However, in other embodiments, the retainer may be separate/distinct from the body 120. The projection(s) may be resiliently biased inwardly (e.g. self-biased), and be temporarily displaceable outwardly to permit passage of the hook 34 and the chordae. For example, the projection(s) may fold back into clearances in the wall of the body 120. Resilient biasing can avoid needing an external influence to move the projections inwardly. In other examples, the projection(s) may be plastically deformable to move from an outward configuration to an inwardly projection configuration, using an externally applied deformation force.

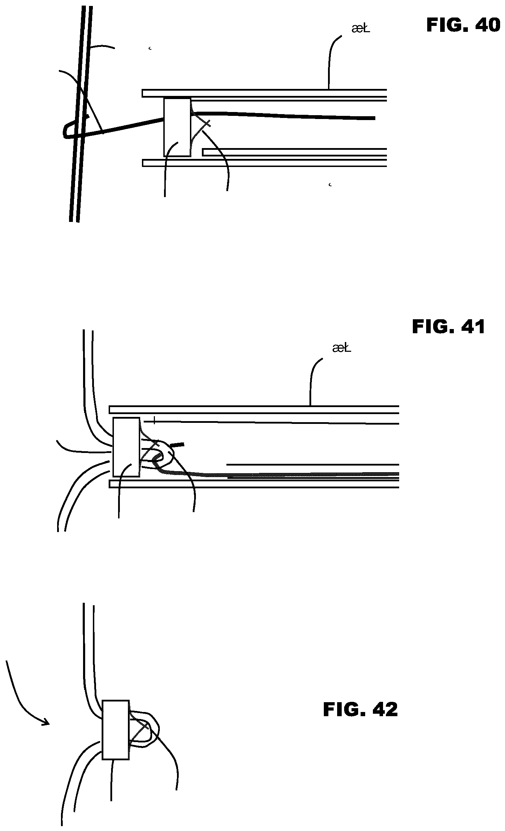

In use, referring to Figs. 40, 44 and 46, a catheter hook 34 (as described previously) is positioned to extend through the implant body 120, or is extended to pass through the implant body 120. The retainer 90 can displace outwardly to accommodate passage of the hook 34, for example, by being pushed outwardly against its resilient bias. The apparatus to manipulated to engage a target chordae with the hook 34. Retracting the hook 34 (e.g. Fig. 41) while the body 120 is restrained by the catheter 32 pulls a portion of the chordae through the mouth 122 of the body 120, and into and/or through the interior space bounded by the body 120, to the retainer 90.

The retainer 90 serves to prevent the pulled-in (or pulled-through) portion of the chordae 20 from pulling-out through the mouth 122 when the hook 34 is disengaged from the chordae and removed (e.g. Figs. 42 and 45). The chordae 20 passes around the projection(s), and the tight bend in the chordae is retained behind the projection(s).

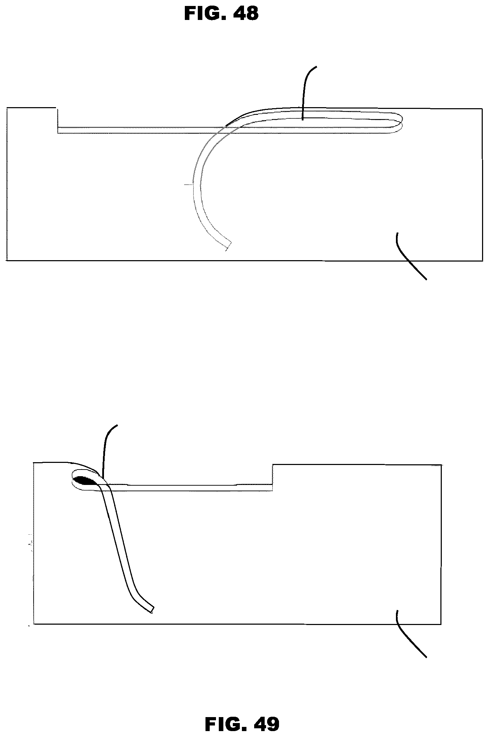

The retainer may optionally comprise a single projection 90 (e.g. Figs. 48 and 49) positioned towards the mouth 122 or towards the opposite end of the implant body 120. The single projection is dimensioned to extend substantially across the space bounded by the body 120. Alternatively, the retainer may comprise plural projections (e.g. first and second projections 90a, 90b shown for example in Figs. 38 and 39). The projections 90a, 90b collectively define an obstruction in or across the spaced bounded by the body 120. Where two projections 90a and 90b are used, the projections may collectively extend generally diametrically. Where three or more projections are used (not shown), the projections may collectively form a star (e.g. a tristar configuration).

Optionally, the distal ends of multiple projections 90a and 90b may have inter-fitting profiles 124a and 124b, to mutually guide one another and/or reinforce engagement between the distal ends of the projections. The inter-fitting profiles may, for example, include a male and female profile shapes.

Howsoever many projections are used, the projections may be generally straight, or curled, or otherwise curved along a portion of their extend. A curved shaped may avoid stress concentrations, and/or enhance the strength and resilience of the projections.

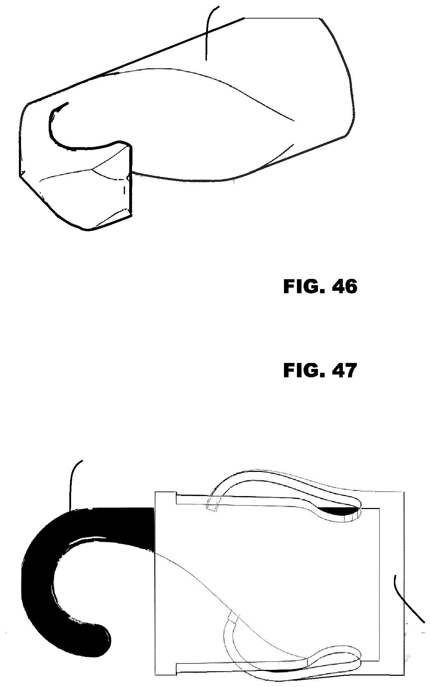

The hook 34 may have any desired form. Two preferred forms are illustrated in Figs. 38-47. In Figs. 38 to 45, the hook 34 has a relatively thin profile, such as a wire-hook. A thinner profile avoids the need for the retainer to displace significantly, and may be preferred for some implementations. In Figs. 46 and 47, the hook 34 has a thicker, crochet-hook profile. The crochet-hook profile has an atraumatic nose, and a generally uniform diameter, reducing the exposure of the free edge of the hook, which may be advantageous in some implementations. Further examples of hook are described later.

The orientation of the hook 34 with respect to the retainer 90 can be chosen to (i) facilitate the hook passing through the retainer 90, and/or (ii) to facilitate engagement of a hooked chordae by the retainer. For example, the hook 34 may be orientated with a major plane parallel or perpendicular to an axis of the retainer 90.

The configurations of retainer 90 described above may also be used in the twelfth example to replace directly the projection 90 of that example.

In the sixteenth to twentieth examples described above, the retainer 90 of the implant 30 is distinct from the hook 34 used to pull the chordae into and/or through the implant 30, the hook 34 being, for example, part of the catheter rather than the implant. In contrast, the twenty-first and twenty-second examples described below, illustrate a hook 34 that also functions as a retainer as part of the implant 30. The implant may thus comprise the hook 34 and the body 120, optionally as two separate elements.

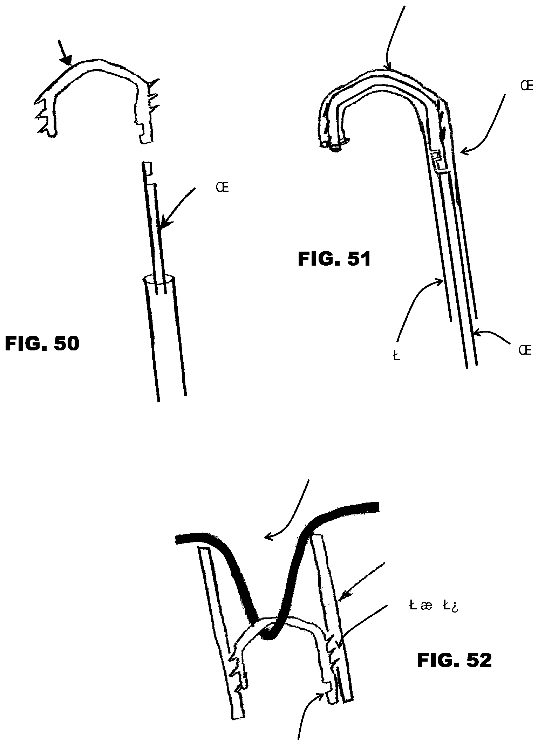

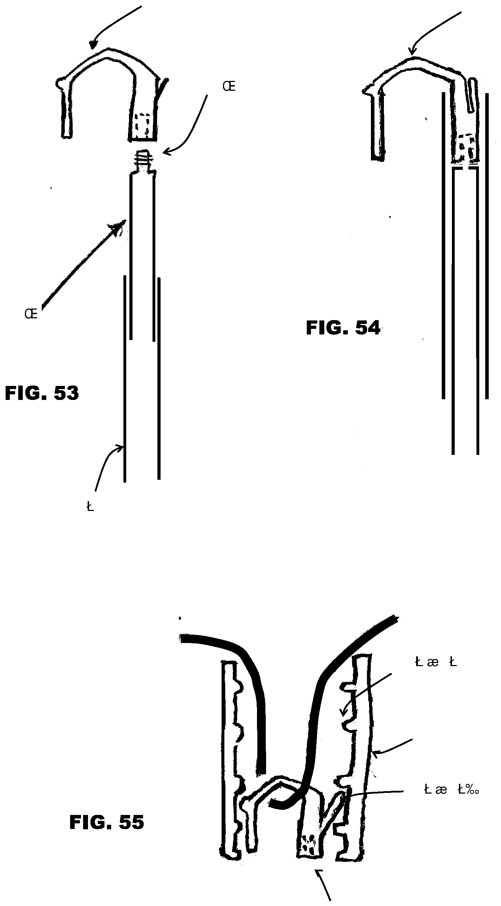

In more detail, in the twenty-first and twenty-second examples, the hook element 34 is coupled via a detachable coupling 126 to a manipulation shaft 36 of the delivery catheter 32. In the example of Figs. 50-52, the detachable coupling 126 is formed by inter-engaging profiles that are held together by relatively close-fitting oversheath 38. While the oversheath 38 overlaps the region of the coupling, applied force (e.g. axial forces and/or torsion) can be transmitted from the shaft 36 to the hook 34. Retraction of the oversheath 38 allows the hook 34 to separate (e.g. permanently) from the shaft 36. In the example of Figs. 53-55, a screw threaded connection forms the detachable coupling 126. A retractable oversheath 38 prevents accidental unscrewing of the detachable coupling 126 until the oversheath 38 is withdrawn. Other types of detachable coupling (e.g. jaws and nipple; or hook) are also described herein.

The position of the detachable coupling 126 is illustrated to be to one side of the hook 34, but this is merely by way of example, and depends on the type of hook 34 used. In the case of a crochet-style of hook 34, the detachable coupling may be towards the centre, for example.

The hook element 34 is initially positioned ahead of the mouth 122 of the implant body 120. For example, the hook element 34 may be pre-positioned in front of the mouth 122. Alternatively, for example, the body 120 may initially be axially separated from the hook 34, and be advanced over the shaft 34 towards the hook element at an appropriate moment. In a similar manner to that described above, after having positioned the hook to engage a native chordae, the hook 34 is retracted relative to the body 120 by relative movement therebetween (e.g. by pulling back on the shaft 36 and/or advancing the body 120). The hook element thus draws a portion of the chordae into a circuitous path within the interior of the body 120.

A retaining mechanism 128 allows the retraction of the hook element 34 into the body 120, but prevents the hook element 34 from advancing out of the mouth 122, thereby prevent pulling-out of the pulled-in portion of the chordae. The retaining mechanism 128 may comprise a ratchet configuration that allows one-way relative movement between hook-element 34 and the body 34, relative movement in the opposite direction being obstructed. In the example illustrated in Figs. 50-52, the retaining mechanism comprises barbs 128a on (at least) one of the hook element 34 and the body 120 that project in a direction to embed in the opposite surface achieve the one-way relative movement. In the example illustrated in Figs. 53 to 55, the retaining mechanism 128 comprises one or more stops 128b formed on (at least) one of the hook element 34 and the body 120 that are engaged by a projection 128c formed on (at least) the other of the hook element 34 and body 120. The projection 128c is configured to ride over and lodge behind a stop 128b.

Once the hook element 34 has been pulled to the desired position within the body 120 and retained by the retaining mechanism 128, the hook 34 may be detached from the catheter 32. The implant 30, composed of the hook 34 lodged in the body 120, remains attached to the native chordae, while the catheter 30 is removed.

The retaining means 128 may define a single position at which the hook element 34 is retained, or it may define plural possible retaining positions. Plural retaining positions may be either: predefined positions, for example, defined by multiple different axial positions of the stops 128c; or a variable position defined, for example, by the barbs 128a which can embed at any position along the opposing surface. Depending on the implementation, a single retaining position may be preferred for simplicity of implantation and/or to achieve an axially short body 120, and/or to achieve a uniform degree of shorting. Plural retaining positions may be preferred for an implementation that allows an operator to choose or control the degree of shortening achieved by a single implant.

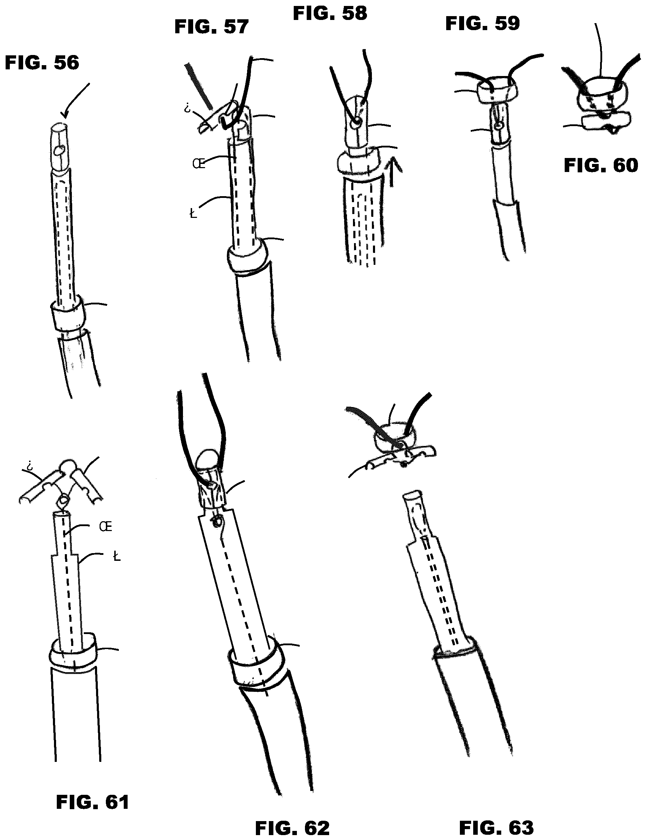

Figs. 56-63 illustrate twenty-third and twenty-fourth examples in which a hook element 34 (also referred to as a puller element) serves as a retainer of the implant. In these examples, the puller element 34 is configured to expand and/or open to a size large than the interior space of the implant body 120, for hooking a native chordae, and to close and/or collapse to a smaller size for passing into and/or through the implant body 120.

The puller element 34 comprises first and second jaws 130a, 130b coupled (e.g. hinged) together at one end. In a diverging configuration (e.g. Figs. 57 and 61), the jaws 130a and 130b define an open hook configuration for hooking a native chordae. In a closed configuration (e.g. Figs. 58, 59 and 62), the jaws 130a, 130b define a closed hinge, or puller, configuration for pulling the chordae into and/or through the implant body 120. At least one of the jaws 130a, 130b may optionally have at least one recess 132 for channelling the chordae therethrough when the jaws are closed. Initially, the puller element 34 is coupled to first and second shafts 36 and 38 of the catheter. One shaft can retain the puller element 34, and the other shaft can change the configuration of the puller element.

In the example of Figs. 56-60, the jaws 130a and 130b are biased closed. Catheter shaft 36 is configured to open the jaws by being advanced from within to bear on at least one of the jaws. After hooking a native chordae, the shaft 38 is retracted to allow the jaws to close and capture the chordae. The puller element 34 and the implant body 120 are moved relative to one another to pull the puller element 34 and the chordae through the implant body from one side to the other. Before being detached from the catheter, the puller element 34 rotates, or is rotated, to extend transverse to the implant body 120, thereby trapping the puller element 34 with the chordae pulled-through the implant body in a circuitous path, in a similar manner to a transverse pledget.

In the example of Figs. 61 to 63, the jaws 130a, 130b are biased open, and are retained initially within a sleeve of catheter shaft 38. The jaws 130a, 130b diverge open when released from the sleeve, to form an open hook. The jaws 130a and 130b are controlled by tension in a control cable 36 which can collapse the jaws 130a and 130b in response to applied tension. Once the chordae has been hooked by the jaws, the jaws 130a and 130b are collapsed closed, and the puller element 34 is pulled through the implant body 120 from one side to the other, in a similar manner to that described above. Upon release from the catheter, the jaws 130a and 130b spring open to define a transverse configuration thereby trapping the puller element 34, and the chordae that has been pulled through the implant body 120.

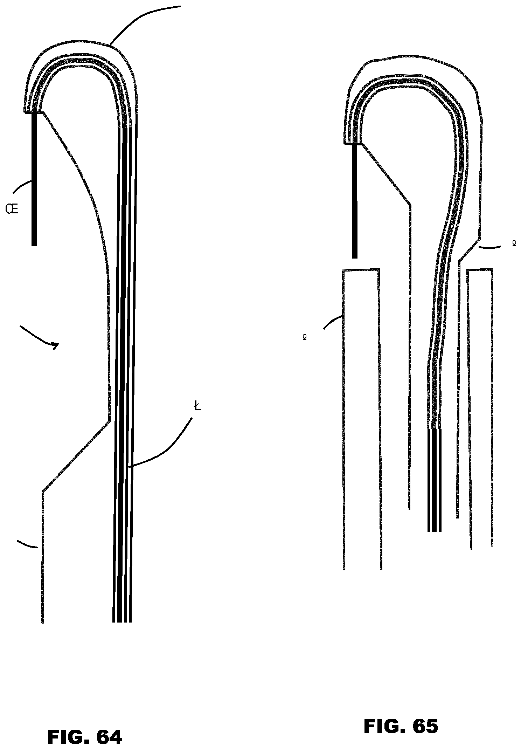

Figs. 64 to 67 illustrate additional configurations of hook and/or puller element 34 usable in any of the preceding embodiments, where appropriate. Referring to Fig. 64, in a twenty-fifth example, the hook element 34 comprises a shaft 140 with an atraumatic tip 142, and an accommodation cavity 144 near the tip 142, in a similar manner to a crochet-style of hook. The cavity 144 has a tapered profile at at least one end, optionally both ends. A tapered profile allows a chordae to be released substantially unhindered from the cavity 144 when the cavity 144 is open. A movable gate portion 146 is displaceable to at least partly extend across the mouth of the cavity 144. The movable gate portion 146 may, for example, have the form of a relatively narrow bar or wire, that slides within a channel 148 within the shaft 140. For example, advancing the wire advances the gate portion 146 across the mouth of the cavity 144; retracting the wire retracts the gate portion. In the current example, the gate portion 146 can project from, and retract into, the distal end of the shaft 140. This enables the element 34 to define a hook configuration, for hooking a native chordae in a similar manner to the examples described above. This is facilitated by the channel 148 extending in a U-shape within the tip 142. The wire or bar is sufficiently flexible to follow the U-shape path, while remaining sufficiently stiff to provide an effective gate portion 146 at its exposed end. The wire of bar may be pre-shaped so that, after traversing the U-path, the wire returns to a straight configuration. For example, the wire or bar may of shape-memory material, for example, nitinol.

As a modification of the above, the tip 142 may be omitted, and the hook shape may instead be defined by a pre-shaped bar or wire 146 that is advanceable from the shaft 140, and adopts its own U-shape once free of the constraining shape of the channel 148. As above, the wire or bar may be of shape-memory material, for example, nitinol.

Fig. 65 illustrates a twenty-sixth example similar to the twenty-fifth example, except that an additional sheath or sleeve 150 is slidable over the shaft to at least partly cover the cavity. A stop 152 may limit the extent to which the sleeve 150 can be advanced over the cavity. The sleeve may enable the puller 34 to have a more uniform external dimension (e.g. diameter), despite the presence of the accommodation cavity 144.

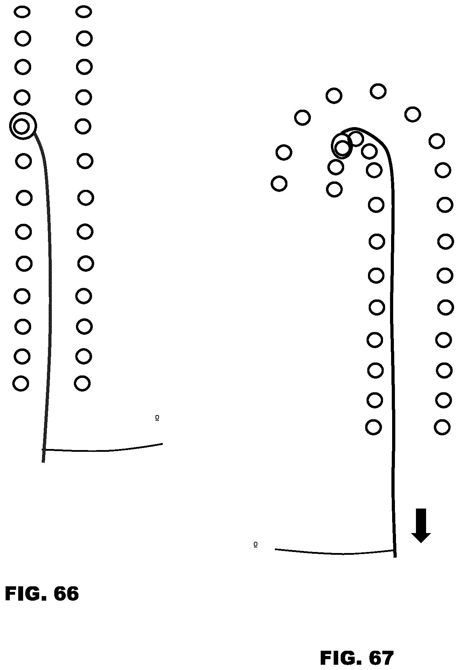

Figs. 66 and 67 illustrates a twenty-seventh example in which a hook element 34 is made of a flexible coil, or segments coupled together. The coil/segments define a hollow flexible body or assembly which, for example, may have a generally straight configuration when relaxed, or a loosely curved configuration. A tensioning member 154 extends within (and/or optionally outside) the body and is coupled to one or more of the segments or coil loops. When tension is applied via the member 154, the segments or coil loops are compressed and adopt a predetermined hook shape. For example, the tensioning member 154 is attached to one side of the segments or coil loop, so as to induce compression on that side, to form the inside curve of the hook shape, without hindering the other side forming the exterior curve of the hook shape. In the illustrated form, the segments or coil loops have a uniform cross-section size and shape. However, in other forms, the segments or coil loops may have an asymmetric form, that is thinner on one side and/or that promotes relative assembly into a curved shape when the segments or coil-loops are urged tightly against one another.

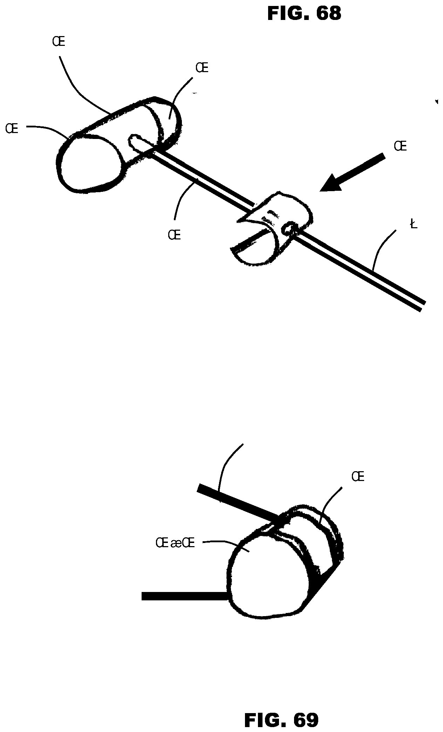

Figs. 68 and 69 illustrate a twenty-eighth embodiment in which the implant 30 comprises a hook element 34 in the form of a rounded, e.g. generally cylindrical, hub 160 defining a waist region between enlarged ends or ridges 162. The hook element 34 is coupled to a catheter shaft 36 by a detachable coupling (not shown) which may, for example, be similar to the detachable couplings 126 described above. A concave clamp element 164 is advanceable over the shaft 36 by means of a pusher shaft 38 towards the hub 160. The clamp element 164 is configured to form an elastic snap-fit around the hub 160.

In use, the hub 160 is positioned spaced apart from and in front of the clamp 164. The hub functions as hook element 34 for hooking a native chordae. Once hooked, the ridges 162 stabilise the chordae on the hub 160, preventing the chordae from slipping off. The shaft 36 is then retracted, and/or the shaft 38 advanced to move the clamp 164 and hub 160 relative to each other, until the clamp 164 clips around the hub 160 to retain the chordae in a generally curved circuitous path around the hub 160 (eg. Fig. 69). Thereafter the catheter shafts are detached from the hub 160 and clamp 164 to leave the assembled implant in situ.

Although in this example, the clamp 164 is biased to form a snap fit around the hub 160, in other examples, the clamp 164 may initially be larger than the hub 160, and may be plastically deformed around the hub 160, for example, by jaws of the catheter (not shown).

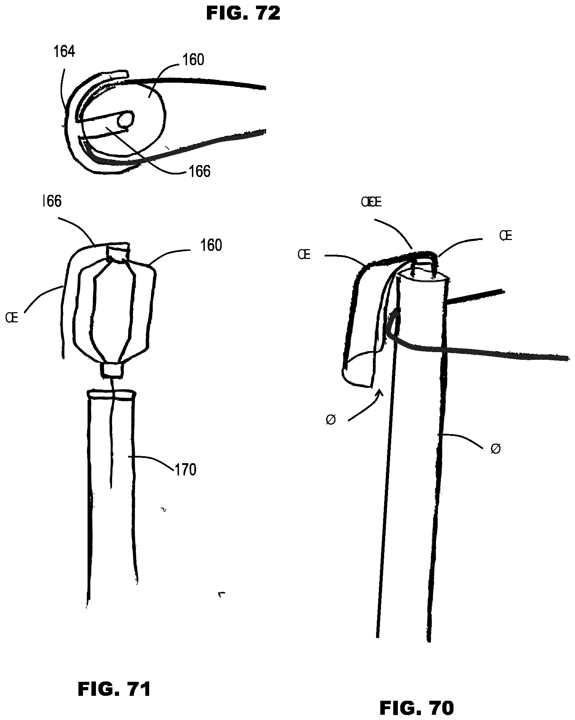

Figs. 70-72 illustrate a twenty-ninth example also comprising a hub 160 and a concave clamping surface 164. In this example, the concave clamping surface 164 is permanently attached via connecting arm 166 to one end (e.g. a distal end) of the hub 160. The hub 160 is reversibly collapsible from an expanded configuration (e.g. Fig. 71) to a collapsed configuration (e.g. Fig. 70), by sliding a constraining sheath 170 over the hub 160. In the collapsed condition, the hub 160 is spaced away from the clamping surface 164, leaving a clearance 172 for hooking a native chordae 20. The hub 160 is expandable to the expanded configuration when the sheath 170 is retracted to expose the hub 160. For example, the hub 160 may be self-expandable, and made from an elastic material. The material may, for example, be a shape memory material, such as nitinol. The hub 160 may optionally have a wireform frame construction to facilitate collapsing and expansion.

In the expanded configuration (e.g. Figs. 71 and 72), the hub 160 expands towards, and optionally against, the concave clamping surface 164, thereby clamping the native chordae in a circuitous path between the hub 160 and the clamping surface 164.

Although in this example, the hub 160 is self-expanding from the collapsed configuration, in other examples, the hub 160 could be of plastically expandable material, and the catheter may comprise a forcible expansion member, for example, an inflatable balloon, for plastically expanding the hub 160.

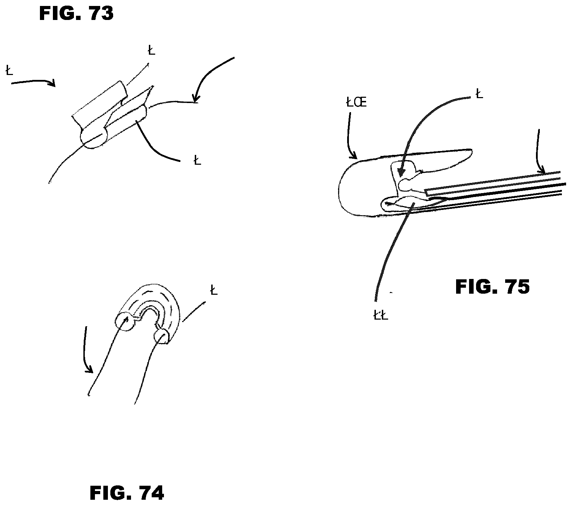

Figs. 73 to 75 illustrate a thirtieth example in which the implant 30 comprises a hollow staple 180. Referring to Fig. 73, the staple 180 may initially have an open channel shape, for example, a rounded channel base 182, and projecting wings 184 at the channel mouth. Additionally or alternatively, the staple may initially be generally straight. Such configurations permit a native chordae 20 to enter easily into the open staple 180. Thereafter, referring to Fig. 74, the staple 180 is deformed to close the channel and to form a curved shape defining a circuitous path for the chordae. Other types and shapes of staple 180 could also be used, as desired.

Fig. 75 illustrates a stapler unit 186 integrated at the tip of a delivery catheter 30, for delivering and fitting the staple 180. The stapler 186 has the form of a hook, and supports the staple 180 in the hollow of the hook shape, with the open mouth of the staple facing the open region of the hook, to admit a chordae into the staple by a simple hooking action. The stapler 186 is configured to compress, e.g. crimp the staple 180 closed, and deform the staple 180 into the curved shape, for example, aided by pressure from an inflatable balloon 188. Thereafter, the catheter 30 with the integrated stapler 186 may be removed.

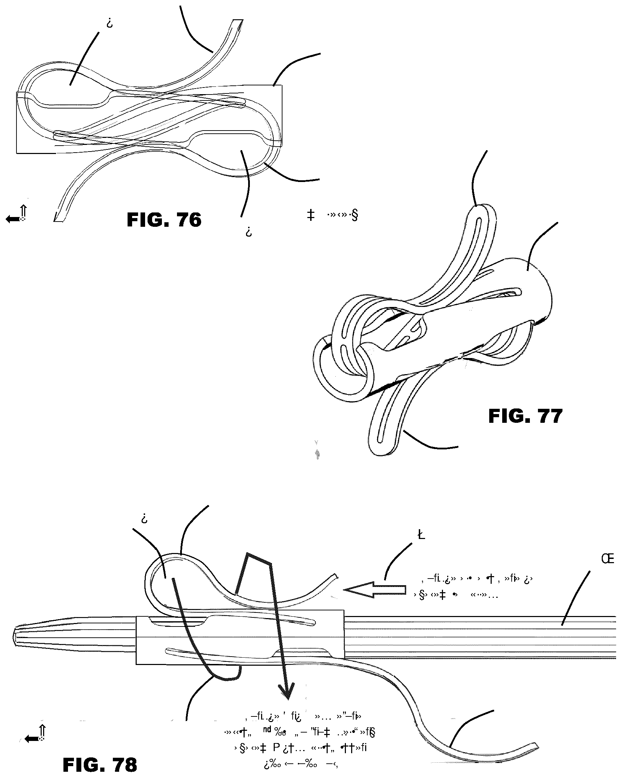

Figs. 76 to 85 illustrate further examples comprising a spool element 200 for winding a portion of the native chordae at least partly around the spool element 200.

Figs. 76 to 78 illustrate a thirty-first example in which the implant 30 comprises a spool element 200 having first and second engagement profiles 202 and 204, for example, arms or lugs. In the illustrated form, the engagement profiles are diametrically opposed to each other. Also in the illustrated form, the engagement profiles extend from opposite ends of the spool element 200 relative to each other. Each engagement profile 202, 204 generally has the form of a resilient S-shaped curve. Each engagement profile 202, 204 is self-biased to a configuration in which the engagement profile defines a generally closed loop around a space 202a, 204a respectively, with the surface of the spool element 200, the engagement profile having a free end that curves away from the spool element 200.

In use one engagement profile (e.g. arm) 202 is used for applying torque to wind (at least partly) a chordae around the spool element 200, and the other engagement profile 204 is subsequently used for retaining the spool in a set rotational orientation with respect to the native chordae tendineae.

Referring to Fig. 78, each engagement profile (e.g. arm) 202, 204 is displaceable, and/or can lift, at least a short distance from the surface of the spool element 200 to permit the native chordae to be guided into the space 202a, 204a defined by the respective engagement profile. In such a state, the engagement profile may act as a hook element. A delivery catheter 32 may include one or more shafts 206 that bear against the engagement profiles 202, 204 from within the spool element 200, and lift the engagement elements as described above. The engagement profiles may have a shape that sits slightly radially below the level of the surface of the spool element 200, and be displaced radially outwardly by one or more shafts advancing through the interior of the spool element 200. The shaft(s) 206 may act on the engagement profiles 202, 204 together, or separate shafts or shaft parts may act on a respective engagement profile 202 or 204 selectively or individually. For example, plural shafts may be nested together or may extend side-by-side.

Fig. 78 illustrates a technique for implanting the spool element 200. With the first engagement profile 202 lifted slightly, the catheter is manipulated to hook a chordae under the free edge of the first engagement profile 202, and into the space 202a, as depicted by arrow 208. Thereafter, the catheter is manipulated to turn and/or rotate the spool element 200, for example, through one or more rotations. The chordae trapped by the first engagement element winds at least partly around the spool element 200. When a desired degree of shortening of the chordae has been achieved, the second engagement profile 204 is manipulated to engage and trap the chordae within the space 204a. This stabilizes the spool element on the chordae, and prevents the chordae from unwinding from the spool element 200.

In the example shown in Fig. 78, the second engagement profile may optionally be folded back to be clear of the spool element 200. This can reduce the risk of the second engagement profile interfering with winding of the chordae around the spool element 200. For example, the second engagement profile 204 may be held in the folded back position by a restraining sheath (not shown). When the sheath is retracted, the second engagement profile 204 is free to spring back to its normal position biased towards the spool element 200.

Although the engagement profiles are illustrated to have a generally curved profile, many other shapes of engagement profile may be used as desired.

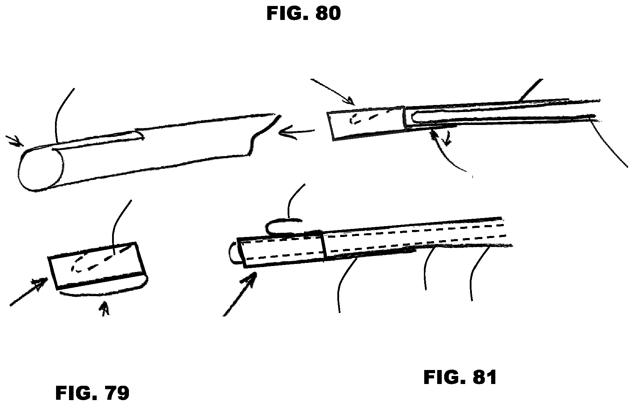

Figs. 79 to 81 illustrate a thirty-second example of implant in the form of a spool element 200 including first and second engagement profiles 202 and 204 having similar functions to those described above. However, in the current example, the first engagement profile is biased to a recessed position, as shown in Fig. 79 in which the engagement profile does not project substantially outwardly of the spool element 200.

Referring to Fig. 80, a delivery catheter comprises a control sheath 210 and an inner pusher shaft 214 that is slidable within the sheath 210. In use, the spool element 200 is loaded at the distal end of the control sheath, with the first engagement profile 202 aligned with an aperture 212 of the sheath, and with the second engagement profile 204 folded back away from the spool element 200. The sheath 201 restrains the second engagement profile 204 in the folded back condition.

In order to deploy the implant, the pusher rod 214 is advanced into the interior of the spool element 200, thereby forcing the first engagement profile to expand outwardly through the aperture 212 of the sheath (as shown in Fig. 81). In this configuration, the first engagement profile 202 can be hooked to a native chordae, and the assembly turned one or more times to wind the native chordae at least partly around the spool element 200. Once the chordae has been shortened by a desired amount, the sheath 210 is retracted, releasing the second engagement profile 204 to fold towards the spool element 200 to its usual position (e.g. Fig. 79). The pusher shaft 214 is then retracted. The first engagement profile 202 biases inwardly into the interior of the spool element, thereby securely anchoring the chordae to the spool element 200, while the second engagement profile stabilizes the spool element with respect to the other side of the chordae, and prevents unwinding of the chordae from the spool element 200.

Figs. 82 to 85 illustrate a thirty-third example that is very similar to the thirty-second example described above, except for the implementation of the second engagement profile 204. Where features are the same or similar, such features are not described again, and reference is made instead to the foregoing description.

In the current example, the second engagement profile 204 is a separate element distinct from the spool element 200. The second engagement profile 204 is shaped as a clip or clamp that forms a snap fit around the spool element 200 after the chordae has been at least partly wound around the spool element 200. The second engagement profile 204 is initially carried outside the control sheath 210, spaced from the spool element 200. Referring to Fig. 84, once the chordae has been shortened by a desired amount, a pusher sleeve 216 advances the second engagement profile 204 towards the spool element 200, until the second engagement profile 204 clips around the spool element 200 with a snap fit. Referring to Fig. 85, once assembled to the spool element 200, the second engagement profile 204 tightly anchors the chordae 20, and stabilizes the spool element 200 against rotation with respect to the chordae 20. If desired, the second engagement element 204 may interlock with one or more features of the spool element 200 to define a unitary structure.

If desired, the spool element 200 of any example may optionally be mounted within, and rotate within, a frame or housing (not shown) that remains stationary. Such an arrangement may provide additional protection of the spool element. The frame or housing may also provide some or all of the functionality of the first and/or second engagement profiles 202 and 204. The frame or housing may serve to limit rotation of the spool element 200, for example, in a winding direction only, and prevent counter-rotation in an unwinding direction.

In all of the preceding embodiments, the different parts of the catheter 32 may optionally have a fixed rotational alignment. A fixed rotational alignment may be implemented by, for example, mechanical keying, such as non-round surfaces of nested parts. For example, the nested parts may have an oval, or polygonal (e.g. triangular or square) cross-section shape. Alternatively, where desired, one or more nested parts may be rotatable with respect to each other.

Although some embodiments illustrate a handle 46 on the implant in the form of a hook, and other embodiments illustrate a handle 46 in the form of a nipple that can be gripped by jaws, these example forms of handle may be interchanged in any example above, and other forms of handle are also envisaged.

Although not illustrated specifically in the preceding drawings, any of the above embodiments may optionally comprise at least one surface that comprises and/or is coated with and/or carries (e.g. as a liner), a compressible and/or flowable material able to conform at least partly around the profile of the native chordae while frictionally engaging against the native chordae. Example materials include silicone, polyurethane foam, and biocompatible rubbers. Such an arrangement can provide a secure yet atraumatic engagement with a native chordae.

Figs. 87 and 88 illustrate a liner or component 230 comprising porous material. The same principles may be used with any of the preceding embodiments. The liner 230 may be substantially co-extensive with the implant 30 (e.g. with the length of the implant 30), or it may line only portion of the implant 30 or, in the illustrated example, the liner 230 may extend beyond one or more extremities. This can provide a generally atraumatic entrance and/or exit of the implant 30.

Fig. 86 illustrates steps of a method of using an implant, for example an implant according to any of the above examples, to treat atrioventricular valve prolapse. The method comprises one or more, or any combination of the following steps:
Step S1: observing a beating heart of a patient using medical imaging (for example, trans-oesophageal ultra-sound imaging, which has been shown to provide good image of leaflet motion, and valve condition);
Step S2: determining from the observation at least one target treatment characteristic relating to shortening of at least one native chordae for treating prolapse of one of more leaflets of an atrioventricular valve of the beating heart, (for example, any one or more of: a location of a target chordae to be shortened; a degree of shortening of a target chordae; a position on a target chordae at which to place an implant; a number of chordae on which to place one or more implants; a number of implants to be implanted)
Step S3: introducing a catheter to a target site in the beating heart of the patient for delivering an implant to a native chordae, and
Step S4: operating the catheter to attach an implant to a native chordae to functionally shorten the native chordae.

Additionally or alternatively to the steps S1-S4, in some embodiments, the step of attaching comprises attaching the implant selectively to a primary chord (e.g. a primary native chordae). A primary chord is a chord that extends from a periphery of a valve leaflet, towards a papillary muscle, to control the peripheral edge. By attaching the implant selectively to a primary chord, it is possible adjust the peripheral edge, for example, to reduce or eliminate prolapse.

Additionally or alternatively, in some embodiments, the step of attaching the implant comprises attaching the implant selectively to a secondary chord (e.g. a secondary native chordae). A secondary chord is a chord that extends from a belly portion of a valve leaflet, towards a papillary muscle, to control bulging of the leaflet belly. By attaching the implant selectively to a secondary chord, it is possible to adjust leaflet bulging, for example, to reduce or eliminate prolapse.

Additionally or alternatively, in some embodiments, both a primary and a secondary chord may be treated by attachment of at least one implant (for example, at least one implant attached to both a primary chord and a secondary chord, or multiple implants attached at least one to at least one of a primary chord and secondary chord).

Additionally or alternatively to any of the above, the step of attaching may comprise attaching the implant to a native chordae at a position selected from (i) closer to the papillary muscle than to the leaflet; (ii) closer to the leaflet than to the papillary muscle; or (iii) generally midway between the leaflet and the papillary muscle.

Additionally or alternatively, the method may comprise reinforcing a native chordae of an atrioventricular valve of a heart, by attaching an implant to the native chordae, the implant configured to reinforce a mechanical property of the native chordae by at least one of:
(i) Inducing inflammation of the tissue of the native chordae;
(ii) Inducing thickening of the tissue of the native chordae;
(iii) Inducing ingrowth of tissue into a porous contact surface of the implant.

It will be appreciated that the foregoing description is merely illustrative of preferred examples, and that many modifications and equivalents may be used within the scope and principles of the invention.

## Claims

1. Apparatus for treating prolapse of an atrioventricular valve, the apparatus comprising an implant (30) deliverable by catheter (32), the implant configured to attach to a native chordae tendineae (20) and to shorten the functional length of the native chordae tendineae.

2. Apparatus according to claim 1, wherein the implant (30) is attachable to an individual native chordae tendineae (20) and configured to shorten the functional length of the respective individual chordae tendineae.

3. Apparatus according to any preceding claim, further comprising a catheter (32) for delivering the implant to a target site in the heart, and a hook (34; 52) configured to hook the native chordae tendineae.

4. Apparatus according to claim 3, wherein the hook is configured to pull the native chordae tendineae in a proximal direction with respect to the catheter, and/or to pull the native chordae tendineae through a mouth of the implant into an interior space of the implant and/or through the implant.

5. Apparatus according to any preceding claim, wherein the implant has a contact surface for contacting native chordae tissue, the contact surface comprising porous material (230).

6. Apparatus according to claim 5, wherein the porous material is selected as one or more of: PET, PTFE, ePTFE, fabric, polyurethane, foamed polyurethane.

7. Apparatus according to claim 5 or 6, wherein the porous material has a pore size of less than 1mm, optionally less than 0.8mm, optionally less than 0.7mm, optionally less than 0.6mm, optionally less than 0.5mm, optionally less than 0.4mm, optionally less than 0.3mm, optionally less than or about 0.2mm.

8. Apparatus according to claim 5, 6 or 7, wherein the pore size is selected to promote ingrowth of native chordae tissue into the porous material.

9. Apparatus according to claim 5, 6, 7 or 8, wherein the implant comprises a structural member, the structural member carrying the porous material to provide the contact surface for contacting a native chordae.

10. Apparatus according to claim 9, wherein the porous material is comprised in a component provided at an interior surface and/or exterior surface of the structural member.

11. Apparatus according to claim 10, wherein the component be secured to the structural member by at least one selected from: one or more sutures; adhesive attachment; welding or fusing of the component to the structural member.

12. Apparatus according to claim 5 or any claim dependent thereon, wherein the porous material covers (i) substantially the entirety of the interior and/or exterior surface of the structural member, or (ii) only a portion of the interior and/or exterior surface.

13. Apparatus according to any preceding claim, wherein the implant is configured to contract around a portion of a native chordae.

14. Apparatus according to any preceding claim, wherein the implant comprises a contact surface comprising a compressible and/or flowable material.
